# EUROPEAN PATENT APPLICATION

(11) **EP 3 971 192 A1**
(43) Date of publication of application: **23.03.2022**
(21) Application number: 20196913.6
(22) Date of filing: 18.09.2020
(51) Int. Cl.: C07F 9/09, C07B 59/00, G01N 24/08, C07F 9/10, G01N 33/68

(54) **PHOSPHATIDYLALKANOL HOMOLOGUES HAVING LABELLED MOIETIES**

(71) Applicant: Chiron AS, 7041 Trondheim (NO)
(72) Inventor: LIU, Huiling, NO-7049 Trondheim (NO); GOROVOY, Alexey, NO-7020 Trondheim (NO); BUTTON, Jennifer, Flimwell, East Sussex TN5 7PH (GB); JOHANSEN, Jon Eigill, NO-7092 Tiller (NO)
(74) Representative: Fitzner, Uwe

(57) **Abstract**

Phosphatidylalkanol homologues having isotopically labelled moieties, methods for preparation of isotopically labelled phosphatidylalkanol homologues and uses of isotopically labelled phosphatidylalkanol homologues.

## Description

The present invention is directed to phosphatidylalkanol homologues having isotopically labelled glycerol moieties, methods for their preparation and their uses.

It should be understood that this invention is not limited to the embodiments disclosed in this summary, or the description that follows, but is intended to cover modifications that are within the spirit and scope of the invention, as de-fined by the claims.

In the present application, including the claims, other than in the operating examples or where otherwise indicated, all numbers expressing quantities or characteristics are to be understood as being modified in all instances by the term "about". Accordingly, unless indicated to the contrary, any numerical parameters set forth in the following description may vary depending on the desired properties one seeks to obtain in the compositions and methods according to the present disclosure. At the very least, and not as an attempt to limit the application of the doctrine of equivalents to the scope of the claims, each numerical parameter described in the present description should at least be construed in light of the number of reported significant digits and by applying ordinary rounding techniques.

Also, it should be understood that any numerical range recited herein is in-tended to include all sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10. The terms "one," "a," or "an" as used herein are intended to include "at least one" or "one or more," unless otherwise indicated.

Any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as set forth herein supersedes any conflicting material incorporated herein by reference. Any material, or portion thereof, that is said to be incorporated by reference herein, but which conflicts with existing definitions, statements, or other disclosure material set forth herein is only incorporated to the extent that no conflict arises between that incorporated material and the existing disclosure material.

### Prior art:

Phosphatidylethanols (PEth) are a group of phospholipid homologues. The structure of PEth consists of a glycerol molecule with two fatty acid chains in sn-1 and sn-2 position and with phosphoethanol as headgroup. Typically, the fatty acids have 14 to 22 carbon atoms with zero to six double bonds. The different PEth homologues are named according to the fatty acid chains attached to the glycerol moiety, basically by adding the kind of fatty acids (in lipid number denotation with or without the exact position and configuration of the double bonds), for example PEth-16:0/18:1 would be phosphatidylethanol having a saturated C16 fatty acid chain in sn-1 position and a mono-unsaturated C18 fatty acid chain in sn-2 position (1-palmitoyl-2-oleoyl-sn-glycero-3-phosphatoethanol), while PEth-16:0/18:2 would be phosphatidylethanol having a saturated C16 fatty acid chain in sn-1 position and a di-unsaturated C18 fatty acid chain in sn-2 position.

Phosphatidylalkanols are homologues of PEth in which the alcohol-residue in the headgroup is not specified, but can also be ethanol.

As phosphatidylethanol is an alcohol biomarker formed in the presence of ethanol in the body, it is high interest to have analytical methods available that can identify this compound quantitatively and qualitatively as accurate as possible.

To this end it has been suggested to provide homologues thereof that have deuterated alkanol in the headgroup, for example PEth-d5 having the five hydrogen atoms in the ethanol of the headgroup replaced by deuterium atoms, in order to use them as standards in the analysis. An example for such prior art is WO 2014/178787 A1.

As the demands posed on the analyses are ever increasing, there is still demand for new phosphatidylalkanol homologues.

### Objects of the invention:

It was an object of the present invention to provide novel phosphatidylalkanol homologues having improved properties versus those already known.
It was a further object of the invention to provide novel methods for the preparation of phosphatidylalkanol homologues.

Additionally, it was an object of the present invention to provide for uses of the novel phosphatidylalkanol homologues and those prepared with the methods of the present invention.

Further objects will become apparent to those skilled in the art upon regarding the following disclosure.

### Solution of the Invention:

These and other objects are solved by the matter outlined in the appended independent claims.

Preferred embodiments are outlined in the dependent claims as well as the following description.

### Detailed description:

The present invention is directed to phosphatidylalkanol homologues having isotopically labelled glycerol moieties in which the glycerol moiety is either deuterated, carbon atoms of it are replaced with ¹³C-isotopes or both.

The alkanol in the compounds is particularly selected from the group consisting of methanol, ethanol, propanols, butanols, pentanols, hexanols and heptanols. Particularly preferred compounds of the present invention have ethanol as alkanol residue. Accordingly, particularly preferred compounds of the present invention are phosphatidylethanol (PEth) homologues.

The phosphatidylalkanol homologues of the present invention have glycerol moieties in which
a) one, two, three, four or five hydrogen atoms, particularly five, of the glycerol moiety are replaced with deuterium, or
b) one, two or three, particularly three, carbon atoms of the glycerol moiety are replaced by ¹³C-isotopes, or
c) one, two, three, four or five hydrogen atoms, particularly five, of the glycerol moiety are replaced with deuterium, and one two or three, particularly three, carbon atoms of the glycerol moiety are replaced by ¹³C-isotopes;
   or, in further alternatives,
d) one or more hydrogen atoms of the fatty acid side chains, particularly palmitic acid side chain, are replaced by deuterium, particularly 31 hydrogen atoms are replaced by deuterium, or
e) one to eighteen carbon atoms in the fatty acid side chains, particularly palmitic acid side chain, are replaced by ¹³C-isotopes, particularly four and 16 carbon atoms in the palmitic acid side chain, are replaced by ¹³C-isotopes;
   or, in still further alternatives,
f) any combination of the above described labelling.

A further aspect of the present invention is a mixture of three internal standards of labelled phosphatidylalkanol homologues, particularly as outlined above, and their use as standards for the Multipoint Internal Calibration Method (MCIM).

In the present invention, phosphatidylalkanol homologues are named as usual with the addition that the specific kind of isotopical labelling is added in parentheses after the usual name. For example "-d5 (glycerol-d5)" added after e.g. PEth-16:0/18:1 means that in the glycerol moiety all five hydrogen atoms are replaced by deuterium and, similarly, "-¹³C₃ (glycerol-¹³C₃)" added after e.g. PEth-16:0/18:1 means that all three carbon atoms of the glycerol moiety are replaced with ¹³C-isotopes.

In particular, the present invention is directed to phosphatidylalkanol homologues of the following two general formulae 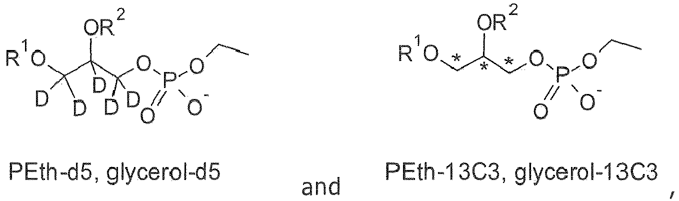 and in further variants also those of the following four formulae, 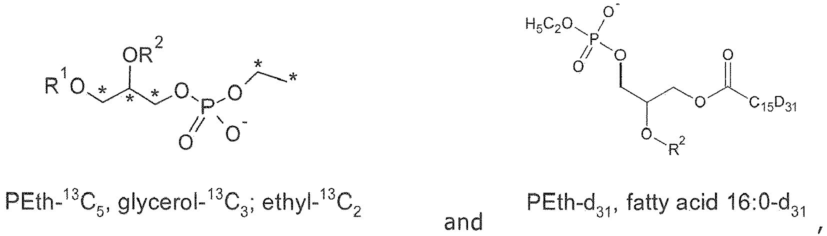 and 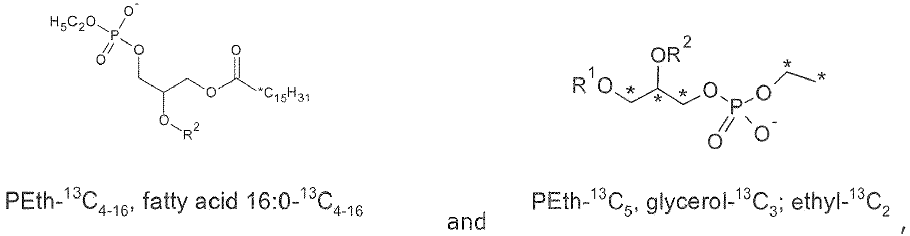 wherein D is deuterium, the asterisk denotes a ¹³C-atom and R¹ and R² are, independently from one another acyl residues having C14 to C22 saturated alkyl chains or C14 to C22 unsaturated alkyl chains with up to seven double bonds. R¹ and R² can be the same or different.

Preferably, R¹ is selected from the group consisting of: acyl residues having C14 to C22 saturated alkyl chains, preferably C12 to C18 saturated alkyl chains, more preferably C15 to C18 saturated alkyl chains, especially preferred C16 and C18 saturated alkyl chains.
One particular preferred saturated acid to obtain R¹ is palmitic acid (lipid number 16:0). Another particular preferred saturated acid to obtain R¹ is stearic acid (lipid number 18:0).
Preferably, R² is selected from the group consisting of acyl residues having: mono-, di-, tri-, tetra-, penta-, hexa- and hepta-unsaturated chains, preferably C14 to C22 mono-, di-, tri-, tetra-, penta-, hexa- and hepta-unsaturated chains, more preferably C16 to C20 mono-, di-, tri-, tetra-, penta-, hexa- and hepta-unsaturated chains, even more preferably mono- and di-unsaturated chains, especially preferred are C18:1 or C18:2.
One particular preferred unsaturated acid to obtain R² is oleic acid (lipid number 18:1 cis-9). Another particular preferred unsaturated acid to obtain R² is linoleic acid (lipid number 18:2, cis-9, cis-12).

In other embodiments of the invention,
R¹ is selected from the group consisting of acyl residues having: mono-, di-, tri-, tetra-, penta-, hexa- and hepta-unsaturated chains, preferably C14 to C22 mono-, di-, tri-, tetra-, penta-, hexa- and hepta-unsaturated chains, more preferably C16 to C20 mono-, di-, tri-, tetra-, penta-, hexa- and hepta-unsaturated chains, even more preferably mono- and di-unsaturated chains, especially preferred are C18:1 or C18:2, and
R² is selected from the group consisting of acyl residues having: C14 to C22 saturated alkyl chains, preferably C12 to C18 saturated alkyl chains, more preferably C15 to C17 saturated alkyl chains, especially preferred C16 and C18 saturated alkyl chains, with the particularly preferred saturated or unsaturated fatty acids being those named above.

In still other embodiments of the present invention both R¹ and R² are
selected from the group consisting of acyl residues having: C14 to C22 saturated alkyl chains, preferably C12 to C18 saturated alkyl chains, more preferably C15 to C18 saturated alkyl chains, especially preferred C16 and C18 saturated alkyl chains,
or both R¹ and R² are
selected from the group consisting of acyl residues having: mono-, di-, tri-, tetra-, penta-, hexa- and hepta-unsaturated chains, preferably C14 to C22 mono-, di-, tri-, tetra-, penta-, hexa- and hepta-unsaturated chains, more preferably C16 to C20 mono-, di-, tri-, tetra-, penta-, hexa- and hepta-unsaturated chains, even more preferably mono- and di-unsaturated chains, especially preferred are C18:1 or C18:2, with the particularly preferred saturated or unsaturated fatty acids being those named above.

Further, according to some embodiments of the present invention, the combinations of fatty acid residues R¹/R² are selected from the group consisting of the following combinations:
14:0/16:0, 14:0/18:1, 14:0/18:2, 14:0/20:4, 16:0/16:0, 16:0/16:1, 16:0/17:1, 16:0/18:0, 16:0/18:1, 16:0/18:2, 16:0/18:3, 16:0/20:2, 16:0/20:3, 16:0/20:4, 16:0/20:5, 16:0/22:4, 16:0/22:5, 16:0/22:6, 16:1/14:0, 16:1/16:1, 16:1/18:0, 16:1/18:0, 16:1/18:1, 16:1/18:2, 16:1/20:4, 17:0/18:1, 17:0/18:2, 18:0/18:1, 18:0/18:2, 18:0/20:3, 18:0/20:4, 18:0/20:5, 18:0/22:4, 18:0/22:5, 18:0/22:6, 18:1/18:1, 18:1/18:2, 18:1/20:1, 18:1/20:2, 18:1/20:3, 18:1/20:4, 18:1/22:4, 18:1/22:5, 18:1/22:6, 18:2/18:2, 18:2/20:1, 18:2/20:4, 20:1/20:4, 20:2/20:4.

General structures of some preferred deuterated (glycerol-d5) and ¹³C-labelled (glycerol-¹³C₃) PEth homologues as disclosed herein are those of 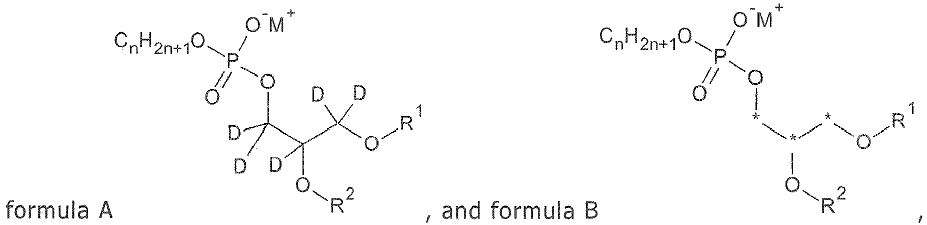 and in further variants also those of the following two formulae, 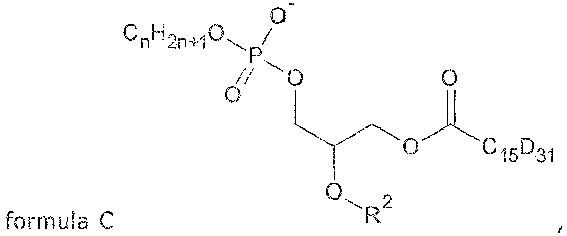 and 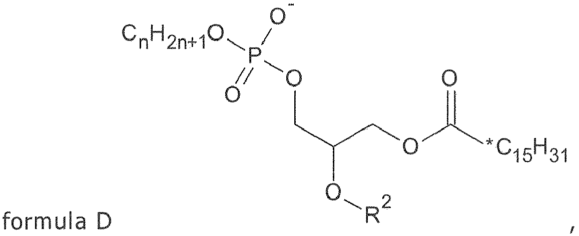 wherein R¹ and R² have the same meaning as above and are fatty acid chains with different number of carbons and different number of double bonds, and M is H, NH₄, Na, K, N(CH₃)₃⁺ and the asterisk (*) represents an ¹³C-atom.
Further, other cations can be used as M in some embodiments of the present invention.
In one preferred variant of the present invention, R¹ is an acyl residue having a saturated alkyl chain and R² is an acyl residue having an unsaturated alkyl chain.

The alkanol in the compounds are particularly selected from the group consisting of methanol, ethanol, propanols, butanols, pentanols, hexanols and heptanols. Particularly preferred compounds of the present invention have ethanol as alkanol residue.

In a preferred embodiment of the present invention,
- all hydrogen atoms in the glycerol moiety are replaced by deuterium atoms, or
- all three carbon atoms in the glycerol moiety are replaced by ¹³C isotopes, or
- all hydrogen atoms in the glycerol moiety are replaced by deuterium atoms and all three carbon atoms in the glycerol moiety are replaced by ¹³C isotopes.

It is particularly preferred that either all hydrogen atoms in the glycerol moiety are replaced by deuterium atoms, or all three carbon atoms in the glycerol moiety are replaced by ¹³C isotopes,

The most preferred compounds according to the invention are 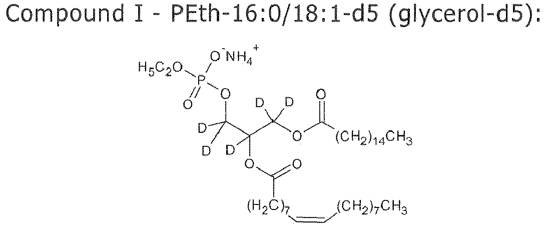 or 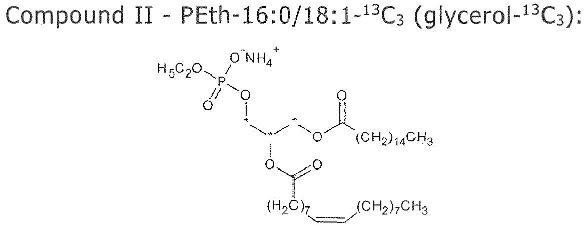 or 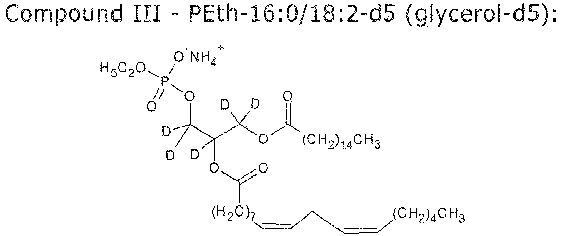 or 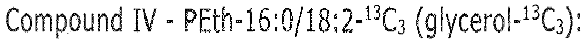 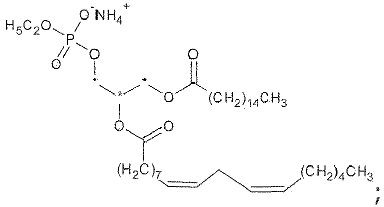 or, in still further aspects,

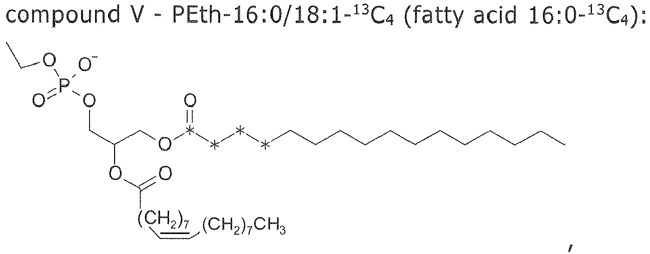

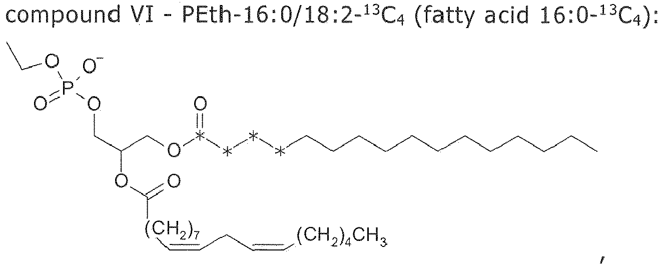

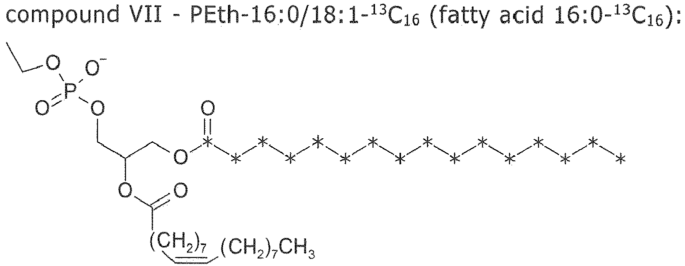

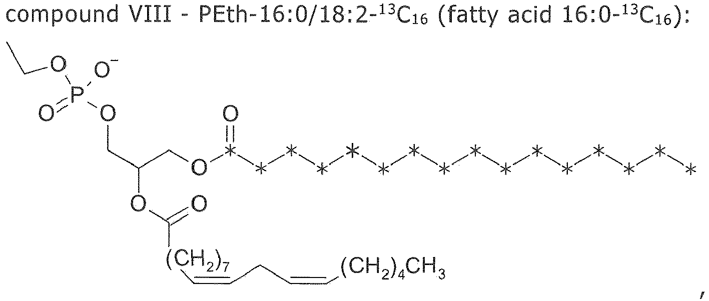

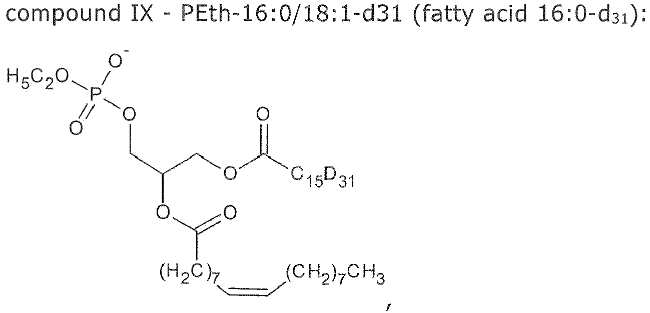

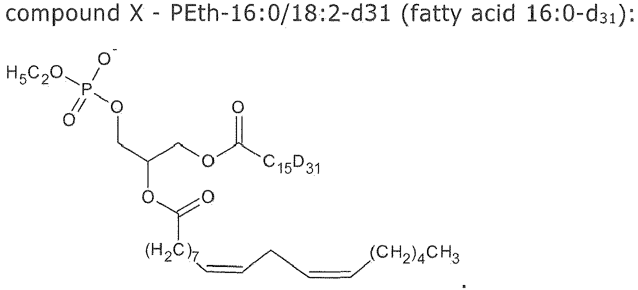

While the first four compounds I to IV are illustrated with ammonia as cation, other preferred compounds of the present invention are those having the same structures but hydrogen, Na⁺, K⁺, N(CH₃)₃⁺ as cations. Similarly, these counterions can be employed for compounds V to X as well.

The four particularly preferred compounds I to IV of the present invention show particularly good properties.

Accordingly, the following compounds are preferred compounds according to the invention, with the first four being particularly preferred:
- PEth-16:0/18:1-d5 (glycerol-d5)
- PEth-16:0/18:1-¹³C₃ (glycerol-¹³C₃)
- PEth-16:0/18:2-d5 (glycerol-d5)
- PEth-16:0/18:2-¹³C₃ (glycerol-¹³C₃)
- PEth-16:0/18:1-d5-¹³C₃ (glycerol-d5 - glycerol-¹³C₃)
- PEth-16:0/18:1-d5-d5-¹³C₃ (ethanol-d5 - glycerol-d5 - glycerol-¹³C₃)
- PEth-16:0/18:1-d5-¹³C₃ (ethanol-d5 - glycerol-¹³C₃)
- PEth-16:0/18:1-d5-d5 (ethanol-d5 - glycerol-d5)
- PEth-16:0/18:2-d5-¹³C₃ (glycerol-d5 - glycerol-¹³C₃)
- PEth-16:0/18:2-d5-d5-¹³C₃ (ethanol-d5 - glycerol-d5 - glycerol-¹³C₃)
- PEth-16:0/18:2-d5-¹³C₃ (ethanol-d5 - glycerol-¹³C₃)
- PEth-16:0/18:2-d5-d5 (ethanol-d5 - glycerol-d5)
- PEth-16:0/18:1-d31 (fatty acid 16:0-d31)
- PEth-16:0/18:2-d31 (fatty acid 16:0-d31)
- PEth-16:0/18:1-¹³C₂ (ethanol-¹³C₂)
- PEth-16:0/18:2-¹³C₂ (ethanol-¹³C₂)
- PEth-16:0/18:1-¹³C₄ (fatty acid 16:0-¹³C₄)
- PEth-16:0/18:2-¹³C₄ (fatty acid 16:0-¹³C₄)
- PEth-16:0/18:1-¹³C₂-¹³C₄ (ethanol-¹³C₂ - fatty acid 16:0-¹³C₄)
- PEth-16:0/18:2-¹³C₂-¹³C₄ (ethanol-¹³C₂ - fatty acid 16:0-¹³C₄)
- PEth-16:0/18:1-¹³C₃⁻¹³C₄ (glycerol-¹³C₃ - fatty acid 16:0-¹³C₄)
- PEth-16:0/18:2⁻¹³C₃-¹³C₄ (glycerol-¹³C₃ - fatty acid 16:0-¹³C₄)
- PEth-16:0/18:1-¹³C16 (fatty acid 16:0-¹³C₁₆)
- PEth-16:0/18:2-¹³C₁₆ (fatty acid 16:0-¹³C₁₆)

Additionally, the following compounds according to the present invention are preferred in some embodiments:
- PMeth-16:0/18:1-d5 (glycerol-d5)
- PMeth-16:0/18:1-¹³C₃ (glycerol-¹³C₃)
- PMeth-16:0/18:2-d5 (glycerol-d5)
- PMeth-16:0/18:2-¹³C₃ (glycerol-¹³C₃)
- PMeth-16:0/18:1-d5-¹³C₃ (glycerol-d5 - glycerol-¹³C₃)
- PMeth-16:0/18:1-d3-d5-¹³C₃ (methanol-d3 - glycerol-d5 - glycerol-¹³C₃)
- PMeth-16:0/18:1-d3-¹³C₃ (methanol-d3 - glycerol-¹³C₃)
- PMeth-16:0/18:1-d3-d5 (methanol-d3 - glycerol-d5)
- PMeth-16:0/18:2-d5-¹³C₃ (glycerol-d5 - glycerol-¹³C₃)
- PMeth-16:0/18:2-d3-d5-¹³C₃ (methanol-d3 - glycerol-d5 - glycerol-¹³C₃)
- PMeth-16:0/18:2-d3-¹³C₃ (methanol-d3 - glycerol-¹³C₃)
- PMeth-16:0/18:2-d3-d5 (methanol-d3 - glycerol-d5)
- PMeth-16:0/18:1-d31 (fatty acid 16:0-d31)
- PMeth-16:0/18:2-d31 (fatty acid 16:0-d31)
- PMeth-16:0/18:1-¹³C₁ (methanol-¹³C₁)
- PMeth-16:0/18:2-¹³C₁ (methanol-¹³C₁)
- PMeth-16:0/18:1-¹³C₄ (fatty acid 16:0-¹³C₄)
- PMeth-16:0/18:2-¹³C₄ (fatty acid 16:0-¹³C₄)
- PMeth-16:0/18:1-¹³C₁-¹³C₄ (methanol-¹³C₁ - fatty acid 16:0-¹³C₄)
- PMeth-16:0/18:2-¹³C₁-¹³C₄ (methanol-¹³C₁ - fatty acid 16:0-¹³C₄)
- PMeth-16:0/18:1-¹³C₃-¹³C₄ (glycerol-¹³C₃ - fatty acid 16:0-¹³C₄)
- PMeth-16:0/18:2-¹³C₃-¹³C₄ (glycerol-¹³C₃ - fatty acid 16:0-¹³C₄)
- PMeth-16:0/18:1-¹³C₁₆ (fatty acid 16:0-¹³C₁₆)
- PMeth-16:0/18:2-¹³C₁₆ (fatty acid 16:0-¹³C₁₆)
In which "Meth" stands for methanol replacing the ethanol in the phosphatidylalkanol homologue.
- PnPr-16:0/18:1-d5 (glycerol-d5)
- PnPr-16:0/18:1-¹³C₃ (glycerol-¹³C₃)
- PnPr-16:0/18:2-d5 (glycerol-d5)
- PnPr-16:0/18:2-¹³C₃ (glycerol-¹³C₃)
- PnPr-16:0/18:1-d5-¹³C₃ (glycerol-d5 - glycerol-¹³C₃)
- PnPr-16:0/18:1-d7-d5-¹³C₃ (n-propanol-d7 - glycerol-d5 - glycerol-¹³C₃)
- PnPr-16:0/18:1-d7-¹³C₃ (n-propanol-d7 - glycerol-¹³C₃)
- PnPr-16:0/18:1-d7-d5 (n-propanol-d7 - glycerol-d5)
- PnPr-16:0/18:2-d5-¹³C₃ (glycerol-d5 - glycerol-¹³C₃)
- PnPr-16:0/18:2-d7-d5-¹³C₃ (n-propanol-d7 - glycerol-d5 - glycerol-¹³C₃)
- PnPr-16:0/18:2-d7-¹³C₃ (n-propanol-d7 - glycerol-¹³C₃)
- PnPr-16:0/18:2-d7-d5 (n-propanol-d7 - glycerol-d5)
- PnPr-16:0/18:1-d31 (fatty acid 16:0-d31)
- PnPr-16:0/18:2-d31 (fatty acid 16:0-d31)
- PnPr-16:0/18:1-¹³C₃ (n-propanol-¹³C₃)
- PnPr-16:0/18:2-¹³C₃ (n-propanol-¹³C₃)
- PnPr-16:0/18:1-¹³C₄ (fatty acid 16:0-¹³C₄)
- PnPr-16:0/18:2-¹³C₄ (fatty acid 16:0-¹³C₄)
- PnPr-16:0/18:1-¹³C₃-¹³C₄ (n-propanol-¹³C₃ - fatty acid 16:0-¹³C₄)
- PnPr-16:0/18:2-¹³C₃-¹³C₄ (n-propanol-¹³C₂ - fatty acid 16:0-¹³C₄)
- PnPr-16:0/18:1-¹³C₃-¹³C₄ (glycerol-¹³C₃ - fatty acid 16:0-¹³C₄)
- PnPr-16:0/18:2-¹³C₃-¹³C₄ (glycerol-¹³C₃ - fatty acid 16:0-¹³C₄)
- PnPr-16:0/18:1-¹³C₁₆ (fatty acid 16:0-¹³C₁₆)
- PnPr-16:0/18:2-¹³C₁₆ (fatty acid 16:0-¹³C₁₆)

In which "nPr" stands for n-propanol replacing the ethanol in the phosphatidylalkanol homologue.
- PiPr-16:0/18:1-d5 (glycerol-d5)
- PiPr-16:0/18:1-¹³C₃ (glycerol-¹³C₃)
- PiPr-16:0/18:2-d5 (glycerol-d5)
- PiPr-16:0/18:2-¹³C₃ (glycerol-¹³C₃)
- PiPr-16:0/18:1-d5-¹³C₃ (glycerol-d5 - glycerol-¹³C₃)
- PiPr-16:0/18:1-d7-d5-¹³C₃ (iso-propanol-d7 - glycerol-d5 - glycerol-¹³C₃)
- PiPr-16:0/18:1-d7-¹³C₃ (iso-propanol-d7 - glycerol-¹³C₃)
- PiPr-16:0/18:1-d7-d5 (iso-propanol-d7 - glycerol-d5)
- PiPr-16:0/18:2-d7-¹³C₃ (iso-propanol-d7 - glycerol-¹³C₃)
- PiPr-16:0/18:2-d7-d5-¹³C₃ (iso-propanol-d7 - glycerol-d5 - glycerol-¹³C₃)
- PiPr-16:0/18:2-d7-¹³C₃ (iso-propanol-d7 - glycerol-¹³C₃)
- PiPr-16:0/18:2-d7-d5 (iso-propanol-d7 - glycerol-d5)
- PiPr-16:0/18:1-d31 (fatty acid 16:0-d31)
- PiPr-16:0/18:2-d31 (fatty acid 16:0-d31)
- PiPr-16:0/18:1-¹³C₃ (iso-propanol-¹³C₃)
- PiPr-16:0/18:2-¹³C₃ (iso-propanol-¹³C₃)
- PiPr-16:0/18:1-¹³C₄ (fatty acid 16:0-¹³C₄)
- PiPr-16:0/18:2-¹³C₄ (fatty acid 16:0-¹³C₄)
- PiPr-16:0/18:1-¹³C₃-¹³C₄ (iso-propanol-¹³C₃ - fatty acid 16:0-¹³C₄)
- PiPr-16:0/18:2-¹³C₃-¹³C₄ (iso-propanol-¹³C₃ - fatty acid 16:0-¹³C₄)
- PiPr-16:0/18: 1-¹³ C₃-¹³C₄ (glycerol-¹³C₃ - fatty acid 16:0-¹³C₄)
- PiPr-16:0/18:2-¹³C₃-¹³C₄ (glycerol-¹³C₃ - fatty acid 16:0-¹³C₄)
- PiPr-16:0/18:1⁻¹³C₁₆ (fatty acid 16:0-¹³C₁₆)
- PiPr-16:0/18:2-¹³C₁₆ (fatty acid 16:0-¹³C₁₆)
In which "iPr" stands for iso-propanol replacing the ethanol in the phosphatidylalkanol homologue.

The present invention is further directed to methods of preparing the disclosed compounds, most particularly PEth-16:0/18:1 and PEth-16:0/18:2 with deuterated or ¹³C-labeled glycerol moiety.

In this context it is to be borne in mind that like naturally occurring phospholipids, most phosphatidylalkanol homologues, particularly PEth homologues, contain unsaturated sn-2-acyl chains possessing from one to four double bonds. Methods for the synthesis of such compounds must be restricted owing to the reactivity of the unsaturated acid side chains and their propensity to undergo facile oxidation. Furthermore, groups that are employed to protect the polar head groups and the phosphate triester must be removable under conditions that do not adversely affect the acyl side chains.
Therefore, the following two methods were found in the context of the present invention:

### Method I:

The synthesis method I includes two parts:
(1) Synthesis of the long-chain mixed acid unsaturated diacylglycerol.
(2) Phosphate coupling of the diacylglycerol with phosphorylation reagent.

This method I is particularly a method for the preparation of isotopically labelled phosphatidylalkanol homologues, comprising or consisting of the following steps:
mI(1) preparation of isotopically labelled 1-alkyloyl-2-alkenoylglycerol comprising or consisting of the steps
   mIa1) providing labelled glycerol in which some or all, preferably all, hydrogen atoms have been replaced with deuterium, or
   mIa2) providing labelled glycerol in which some or all, preferably all, carbon atoms have been replaced with ¹³C-isotopes, or
   mIa3) providing labelled glycerol in which some or all, preferably all, hydrogen atoms have been replaced with deuterium, and some or all, preferably all, carbon atoms have been replaced with ¹³C-isotopes; or
   mIa4) providing unlabelled glycerol
   mIb) protecting two of the OH/OD-groups of the glycerol moiety, preferably under formation of an acetal-structure;
   mIc) protecting the remaining OH/OD-group in the 3-position with a protecting group, preferably a levulinoyl-group;
   mId) deprotecting the first two OH/OD-groups;
   mIe) addition of a C12 to C18 saturated alkyl chain, preferably C15 to C18 saturated alkyl chain, especially preferred C16 and C18 saturated alkyl chain to the sn-1 position of the glycerol moiety;
   mIf) addition of a mono-, di-, tri- and tetra-unsaturated chain, preferably C16 to C20 mono-, di-, tri- and tetra-unsaturated chain, more preferred mono- and di-unsaturated chain, especially preferred are C18:1 or C18:2 to the sn-2 position of the glycerol moiety;
   mIg) removing the second protective group, preferably with hydrazine hydrate;
mI(2) reacting the isotopically labelled 1-alkyloyl-2-alkenoylglycerol product of step (1) with a phosphorylation reagent having
   i) if an unlabelled glycerol is provided in step mIa4), a labelled alkanol group, preferably a labelled ethanol group; wherein all or some of the hydrogen atoms of the alkanol residue are replaced by deuterium and/or wherein some or all the carbon atoms of the alkanol residue are replaced by ¹³C-isotopes, or
      a labelled fatty acid chain, wherein all or some of the hydrogen atoms are replaced by deuterium and/or wherein some or all the carbon atoms of the alkanol residue are replaced by ¹³C-isotopes;
   ii) if a labelled glycerol is provided in steps mIa1), mIa2) or mIa3), an unlabelled alkanol group, preferably an unlabelled ethanol group, wherein no atom of the alkanol residue has been replaced by deuterium and/or ¹³C;
      mIh) optionally removing a protecting agent, if present on the phosphorylation agent;
      mIi) optionally converting the product to a salt-form of the product.

The first part comprises the synthesis of long-chain mixed-acid unsaturated diacylglycerols, particularly via levulinoyl protection.

Starting from labelled glycerol, in which all hydrogens have been replaced with deuterium, mixed-acid, unsaturated 1,2-diacyglycerols, were prepared in pure form via levulinoyl protection. The method is the synthesis of a mixed-acid triacylglycerol, containing levulinoyl in the 3-position as the protective group, followed by selective removal of the latter group by, for example, hydrazine hydrate. The two possible side-reactions - acyl migration and hydrogenation of double bonds were avoided by this method.

Hence, in the present invention's method I the levulinoyl protection method is applied, which is different from the method in WO 2014/178787 A1, in which 4-methoxybenzyl protecting group is used.

1,2-Diacyglycerols are difficult to prepare in the pure form because of the readiness of acyl migration in the 1,2-isomer giving the 1,3-isomer. Temporary protection of the 3-hydroxy group is necessary to prevent acyl migration. Previously, methods used benzyl, methoxybenzyl, tetrahydropyranyl, trityl and 2,2,2-trichloroethoxycarbonyl protection.
For the synthesis of an unsaturated, mixed-acid 1,2-diacylglycerol, the protecting group should be: easy introduction as well as removal, unattended by acyl migration (leading to mixtures of 1,2-/1,3-isomers) or hydrogenation of double bonds.

This way of preparation has some considerable advantages, inter alia, deprotecting is much easier, higher product yield of diacylglycerols are achieved and, most importantly, acyl chain migration was avoided.

The second part is the phosphate coupling or phosphorylation of diacylglycerol with a phosphorylation reagent, preferably a dialkoxy(diaalkylamino)phosphine in which one of the dialkoxy residues can be a protecting/leaving group like a 2-cyanoethyl group. The alkyls attached to the nitrogen can be the same or different and may together form a ring; examples would be methyl, ethyl, n-propyl, iso-propyl, n- or isobutyl or forming a tetrahydrofurane- or morpholine-residue with the nitrogen, especially preferred both are isopropyl.
Particularly preferred as phosphorylation agent is that of formula 8 which may be prepared as in scheme Ia, below.

This phosphorylation can be applied under mild conditions and is based on well-known P(III) chemistry. The protecting group on the phosphoric acid moiety is 2-cyanoethyl group, which is easy to remove under mild conditions.
The phosphorylation reagent 8 is not commercially available, and was synthesized by using the method as shown in Scheme Ia, below.

The following scheme I shows in an exemplary manner the synthesis of PEth-16:0/18:1-d5 (glycerol-d5) via method I.
The first part is the synthesis of 1-palmitoyl-2-oleoylgycerol-d5 (7), while the second part is the phosphate coupling and preparation of PEth analogue and its salt.

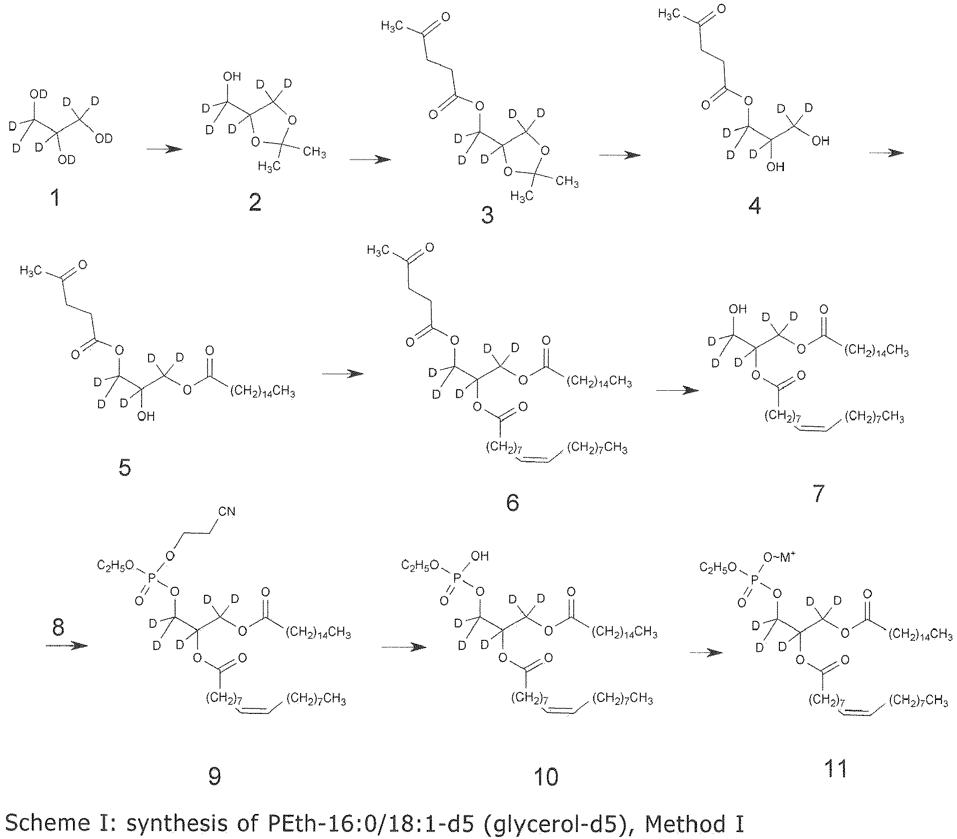

This method I can also similarly be applied in the synthesis of ¹³C₃-labelled PEth by changing the starting material to ¹³C-labelled glycerol.

Of course, this method I is also applicable in the synthesis of other phosphatidylalkanol homologues, particularly other PEth homologues, having different fatty acid chains, as seen in the scheme I-general below. The synthesis includes two parts: (1) Synthesis of the long-chain mixed acid unsaturated diacylglycerol and (2) phosphate coupling of the diacylglycerol with phosphorylation reagent 8. The residues R¹ and R² are as outlined above.

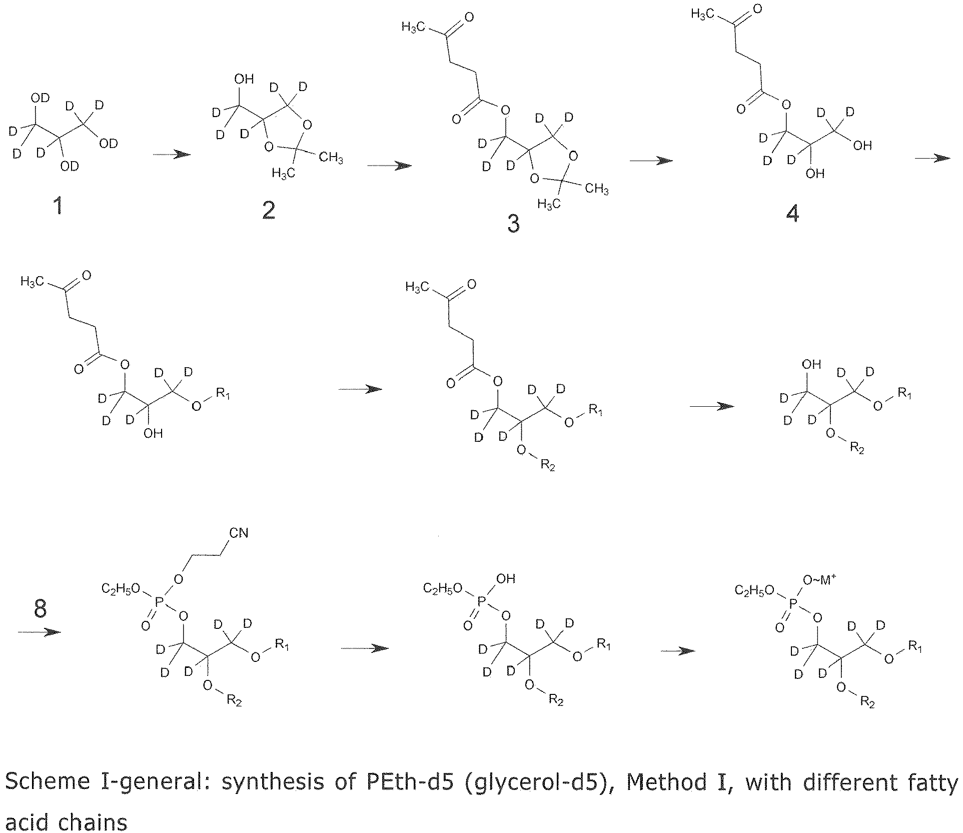

While different phosphorylation reagents may be used in the method I of the present invention, particularly preferred ones according to the following formula 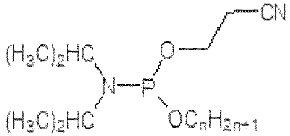 can be prepared according to the following scheme Ia-alkanol:

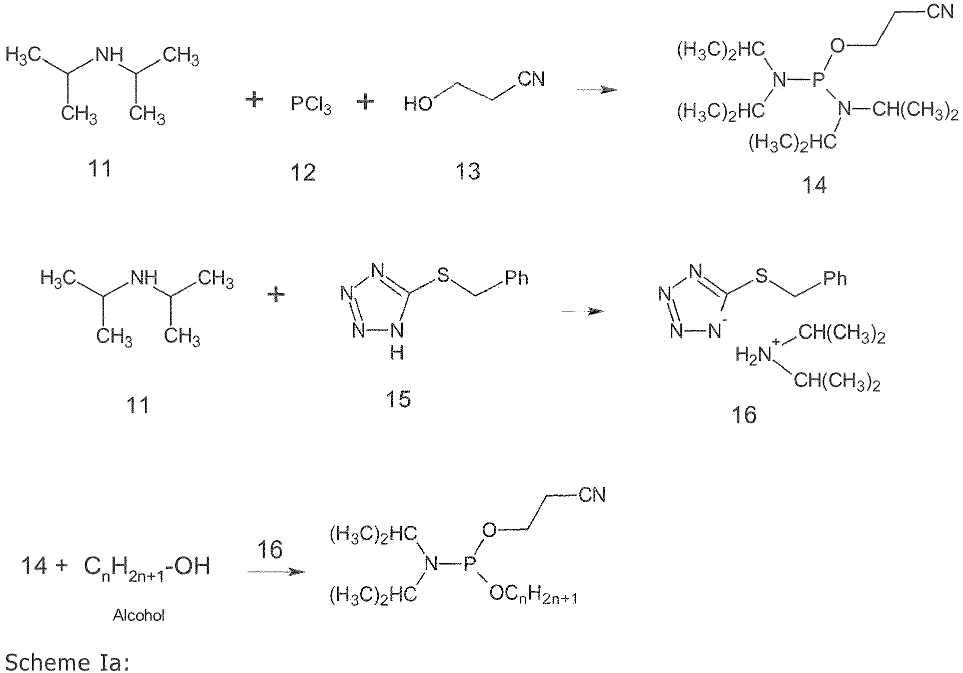

Even more preferably, the phosphorylation agent is one in which the alkanol is ethanol and is prepared according to the following scheme Ia-ethanol:

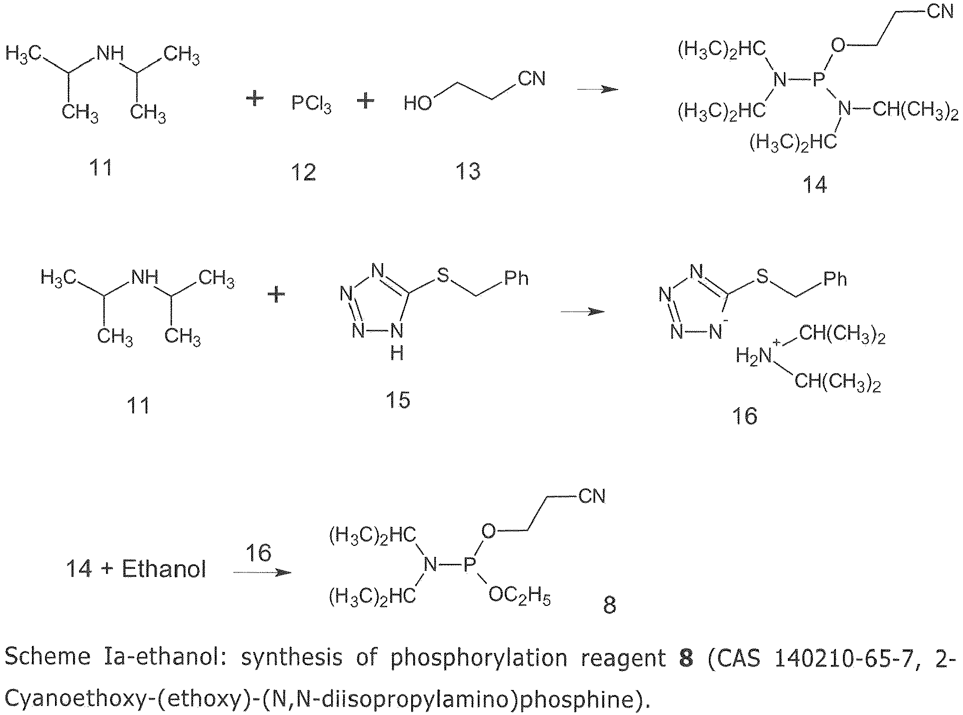

Accordingly, in the context of the present invention a new preparation method for phosphorylation reagent and especially those of a compound of formula 8 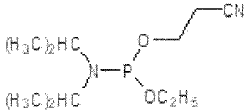 was found, the method comprising or consisting of the steps
aa) providing diisopropylamine and reacting with 2-cyanoethanol and phosphorous trichloride;
bb) providing diisopropylamine and reacting with 5-Benzylthio-1H-tetrazole (CAS 21871-47-6) 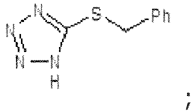
cc) reacting the product from step aa) with alkanol, in particular ethanol, in the presence of product of step bb).

The compound of formula 8 is particularly useful as phosphorylation agent.

### Method II:

This second method is an alternative synthesis method to that of method I and is different from method I in two aspects:
1) The phosphate head group is coupled to a protected 1-monoacyl glycerol, in which the 2-position is protected (cf. formula 21 in scheme II, below) first, and only after that acylation to introduce unsaturated acyl group at the sn-2-position is done in order to avoid any side reactions because of the activities of the double bonds.
2) Alkyl or aryl dichlorophosphite, particularly benzyl dichlorophosphite, is used as phosphorylation reagent instead of the reagent 8.

This method is particularly a method for the preparation of isotopically labelled phosphatidylalkanol homologues, comprising or consisting the following steps:
mIIa1) providing labelled glycerol in which some or all, preferably all, hydrogen atoms have been replaced with deuterium, or
mIIa2) providing labelled glycerol in which some or all, preferably all, carbon atoms have been replaced with ¹³C-isotopes, or
mIIa3) providing labelled glycerol in which some or all, preferably all, hydrogen atoms have been replaced with deuterium, and some or all, preferably all, carbon atoms have been replaced with ¹³C-isotopes; or
mIIa4) providing unlabelled glycerol;
mIIb) protecting two of the OH/OD-groups of the glycerol moiety, preferably under formation of an acetal-structure;
mIIc) protecting the remaining OH/OD-group in the 3-position with a protecting group, preferably 4-methoxybenzyl;
mIId) deprotecting the first two OH/OD-groups;
mIIe) addition of a C12 to C18 saturated alkyl chain, preferably C15 to C17 saturated alkyl chain, especially preferred C16 saturated alkyl chain to the sn-1 position of the glycerol moiety or
   addition of a labelled C12 to C18 saturated alkyl chain, preferred C16 and C18 saturated alkyl chain to the sn-1 position of the glycerol moiety;
mIIf) protecting the hydroxyl-group in 2-position, preferably with benzyloxylmethyl or tetrahydroxy pyranyl;
mIIg) removal of the first protection group;
mIIh) coupling the protected isotopically labelled protected 1-acylglyerol product of step mIIg) with a phosphorylation reagent having
   i) if an unlabelled glycerol is provided in step mIIa4),
      a labelled alkanol group, preferably a labelled ethanol group; wherein all or some of the hydrogen atoms of the alkanol residue are replaced by deuterium and/or wherein some or all the carbon atoms of the alkanol residue are replaced by ¹³C-isotopes,
      or
      a labelled fatty acid, preferably 16:0 and 18:0, is introduced to the sn-1 position;
   ii) if a labelled glycerol is provided in steps mIIa1), mIIa2) or mIIa3), an unlabelled alkanol group, preferably an unlabelled ethanol group, wherein no atom of the alkanol residue has been replaced by deuterium and/or ¹³C;
mIIi) deprotecting the hydroxyl group in 2-position;
mIIj) addition of a mono-, di-, tri- and tetra-unsaturated chain, preferably C16 to C20 mono-, di-, tri- and tetra-unsaturated chain, more preferred mono- and di-unsaturated chain, especially preferred are C18:1 or C18:2 to the sn-2 position of the glycerol moiety;
mIIh) optionally removing a protecting agent, if present on the phosphorylation agent;
mIIi) optionally converting the product to a salt-form of the product.

The following scheme II shows in an exemplary manner the synthesis of PEth-16:0/18:1-d5 (glycerol-d5) via method II.

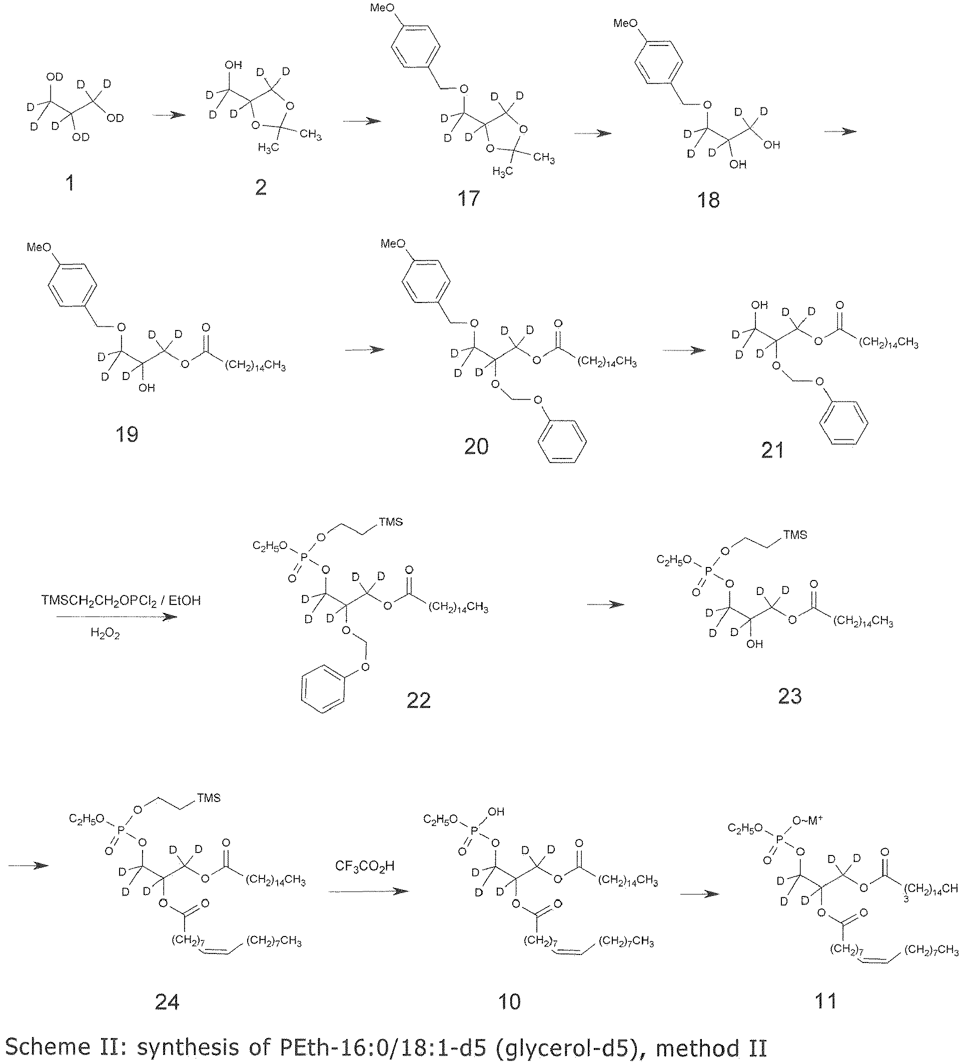

1-monoacyl (saturated chain)-glycerol (19), containing 4-methoxybenzyl as protective group in the 3-position to prevent 1, 3-acyl migration is synthesized. As a different possible protective group benzyloxymethyl (tetrahydroxy pyranyl can also be used. After that protection is added in the 2-position (compound 20) before the 4-methoxybenzyl protective group is removed. The protection in the 2-position is done in order to prevent 1,2-acyl migration. The protected 1-acylglycerol (21) is then coupled with (trimethylsilyl)ethyl dichlorophosphite, followed by oxidation of the glycerolphosphite with hydrogen peroxide (or t-BuOOH) to the phosphate (22).

Removal of the benzyloxy methyl protection group from the sn-2 position of 22 by catalytic hydrogenation without 1,2-acyl or phosphoryl migration. Subsequent acylation of the intermediate (23) with the unsaturated fatty acid followed by deprotection from the phosphate triester (24) gave PEth as free acid, which can be transferred to desired salts.

The phosphite coupling procedure with for example (trimethylsilyl)ethyl dichlorophosphite provides a simplified and more efficient route to protected lyso-PEth that then can be acylated with the unsaturated fatty acids at the sn-2 position. Compared to the reagent 8, which must be synthesized in several steps, alkyl or benzyl dichlorophophites are normally commercially available or easily to be made from PCl₃ in one step reaction.
With (trimethylsilyl)ethyl dichlorophosphite as phosphorylation reagent, the coupling reaction can be conducted with Di(isopropyl)amine, the deprotecting to remove the (trimethylsilyl)ethyl group is easily achieved under mild conditions.

In both above mentioned methods it is possible and preferred to add the saturated and unsaturated fatty acids in the order indicated above. However, it is also possible to change the order of addition of the fatty acids, i.e. it is possible to first add the unsaturated fatty acid and then the saturated one. It is to be noted though, that in method II it is more preferred to keep the order outlined above.

The compounds of the present invention can preferably be used in quantitative and/or qualitative analysis of unlabelled phosphatidylalkanols, especially PEth. In more specific embodiments, the compounds of the invention are used as standards, preferably as internal standards.

The compounds of the present invention can be used for HPLC or LC-MS, particularly as internal standards.

Usually, for qualitative analysis (PEth identification), PEth is analysed by LC-MS/MS mostly. Thereby, the analysis is to detect the molecular ion (m/z) and the assignment of the major products ions of the sn-2 and sn-1 fatty acid chains, e.g. when analysing PEth 16:0/18:1, the 16:0 fatty acid anion with m/z 255 is assigned to the sn-1-position and the 18:1 anion with m/z 281 is assigned to the sn-2 position. In the prior art, native PEth with known structure is used as reference standards for the identification; sometimes in the prior art isotopically labelled PEth-d5 (16:0/18:1 ethyl-d5), in which the ethyl residue of the "ethanol-group" is partly or fully deuterated, is used as internal standard.
The main use of internal standards is to compensate for losses during sample preparation and variable detection sensitivity of the LC-MS/MS system.
This procedure can be enhanced by using the compounds of the present invention, particularly compounds I, II, III and IV, and in further aspects compounds V to X, as internal standards

Similarly, during the LC-MS/MS (or LC-ESI-MS/MS) analysis of PEth homologues monitoring on the major products ions of the sn-2-and sn-1 fatty acid chain, comparing with reference materials (PEth) was the way to work.
Again, this can be enhanced by using the compounds of the present invention, particularly compounds I, II, III and IV, and in further aspects compounds V to X, as internal standards.

Another method/technique of combined ozone induced dissociation MS (OzID) and collision induced dissociation mass spectrometry (CID) can also be used for structural characterization of e.g. glycerophospholipids. The complete structure elucidation by this method is based on the product arising from CID of the [M+ Na]⁺(adduct ions of phospholipids) which can be isolated and subjected to subsequent OzID. The resulting CID-OzID MS yields abundant product ions that are characteristic of the acyl substitution on the glycerol backbone (the sn-position). This method can differentiate regioisomers (sn-position isomers), and also assign double bond positions to individual acyl chains at the specific backbone positions.
Again, this can be enhanced by using the compounds of the present invention, particularly compounds I, II, III and IV as internal standards.

The preparation methods of the present invention are also superior over the prior art in that less impurities are present in the final product if at all, and if so, these are more easily removed than the impurities present when preparing such compounds with methods of the prior art, like the method described in WO 2014/178787 A1.

In methods according to the prior art (e.g. WO 2014/178787 A1) the obtained PEth can contain 2,3-fatty acid migration isomers, which are difficult to separate from the intended product. These are not obtained by the methods of the present invention, or at least to a considerably lesser extent.

One problem of the prior art was that the regio-isomers cannot be separated from PEth in LC-MS/MS analysis, because of the similar chromatographic and spectral properties as well as the identical molecular weight, which made the quantification of PEth homologues inaccurate. With the compounds of the present invention, this is not the case anymore, inter alia because of different molecular weights.

With the methods of the present invention it is possible to prepare highly pure PEth homologues without unwanted regioisomers and without other chemical impurities being present.

The compounds of the present invention, particularly compounds I, II, III and IV, can thus be used as labelled standards, particularly internal standards, for analytical processes and methods.
In preferred embodiments the compounds of the present invention, particularly compounds I, II, III, IV, and in further aspects compounds V to X, are thus used as labelled standards, particularly internal standards, for LC-MS/MS or LC-ESI-MS/MS or CID-OzID MS or HPLC or LC-MS or HPLC or high throughput UPLC^{®}-MSMS.
The compounds of the present invention show several beneficial properties in this regard.

Similarly, phosphatidylalkanol homologues prepared according to method I or method II of the present invention, particularly phosphatidylalkanol homologues with isotopically labelled glycerol moieties, can also be used as labelled standards, particularly internal standards, for analytical processes and methods, especially for LC-MS/MS or LC-ESI-MS/MS or CID-OzID MS or HPLC or LC-MS or HPLC or high throughput UPLC^{®}-MSMS.

The compounds of the present invention can be used in compositions together with various solvents.

Preferred is to use the compounds of the present invention in mixtures with non-deuterated solvents, particularly with acetonitrile, isopropanol, isopropanol, methanol, isooctane or chloroform, more preferred with chloroform, isopropanol, methanol and isooctane, and especially preferred with non-deuterated chloroform.

The phosphatidylalkanol homologues of the present invention, particularly compounds I, II, III, IV, and in further aspects compounds V to X, or phosphatidylalkanol homologues prepared according to the present invention's method I or II, particularly those with isotopically labelled glycerol moieties, can be used as reference materials and internal standards for quantitative analysis of PEth homologues in human blood samples, especially by LC-MS/MS or LC-ESI-MS/MS, or for the study of individual PEth homologues.

The compounds of the present invention, particularly the preferred compounds I, II, III, IV, and in further aspects compounds V to X, have several beneficial properties, for example when compared with known PEth homologues in which the ethanol residue is labelled with deuterium, some of the advantages are:
- deuterium in the backbone of the molecule is more stable than in the side chains (as for example in PEth-d5),
- ¹³C₃-PEth, particularly of formula II, IV, V, VI, VII, and VIII, are more stable than deuterated PEth, deuterium can be exchanged with protons
- ¹³C₃-PEth, particularly of formula II, IV, V, VI, VII, and VIII give rise to less ion-suppression problems in LC-MS analysis,
- they often show improved sensitivity when employed in assays,
- they show improved stability,
- they result in reduced interference when being used in assays and/or as internal standards,
- they can be synthesized more efficiently.

The phosphatidylalkanol homologues with penta-deuterated glycerol moiety described herein have a molecular weight that is about 5 Dalton higher than the corresponding non-labelled, i.e. non-deuterated lipids.
The phosphatidylalkanol homologues with all three carbon atoms in the glycerol moiety being ¹³C isotopes described herein have a molecular weight that is about 3 Dalton higher than the corresponding non-labelled lipids, in which the glycerol moiety has only ¹²C isotopes.

The phosphatidylalkanol homologues with all carbon atoms in one of the fatty acid side chains being ¹³C isotopes described herein have a molecular weight that is about 4 or 16 Dalton higher than the corresponding non-labelled lipids, in which the fatty acid moiety has only ¹²C isotopes

The phosphatidylalkanol homologues with penta-deuterated glycerol moiety and all three carbon atoms in the glycerol moiety being ¹³C isotopes described herein have a molecular weight that is about 8 Dalton higher than the corresponding non-labelled lipids, in which the glycerol moiety has only ¹²C isotopes
These differences enable the use of these compounds as internal standard in analytical applications based on e.g. LC-MS(/MS) or LC-ESI-MS/MS techniques.

The deuterium and ¹³C labelled compounds presented in this invention, possibly in combination with currently known labelled compounds, possibly also with ¹³C2 in the ethyl group, enables the application of a mixture of three or more labelled compounds in the internal calibration method.

In one embodiment, the compounds of the present invention and compositions containing them are not used for or in ¹H-QNMR.
In one embodiment, the compounds of the present invention do not have deuterated alkanol residues.

Upon testing of the compounds of the present invention, particularly compounds I, II, III and IV, it was found that the deuterium in the glycidyl backbone of the molecule is more stable than if present in side ethyl chains:
In experiments no proton-deuterium exchanges and ester exchanges were observed for the compounds of the present invention, particularly compounds I, II, III, IV, and in further aspects compounds V to X, but, on the other hand, by-products were observed by loss of the entire phosphate head group (including the ethyl-d5) from PEth-d5. Similarly, In the method of CID-OzID MS, the abundant product ions are formed after missing the phosphate head as well, so the advantage of labelling in the glycerol backbone according to the present invention is that the labelling is not lost in the analysis.

It was found that the ¹³C-labelled compounds of the present invention, particularly compounds II, IV, V, VI, VII, and VIII, co-elute with the analyte in LC, while deuterated compounds have different retention times from the analyte. Co-elute in this context means that the standards and analyte will have the same matrix effects, ion suppression for example.

It is to be understood that deuterium atoms and ¹³C atoms occur naturally. Accordingly, any reference to replacing (or the like formulations) in the context of the present invention when relating to the atoms of the glycerol moiety or the alkanol moiety are to be understood as intentional replacements and do not take into account possible naturally occurring deuterium or ¹³C-atoms.

The invention is now described in more detail with reference to the following non-limiting examples. The following exemplary, non-limiting examples are provided to further describe the embodiments presented herein. Those having ordinary skill in the art will appreciate that variation of these examples are possible within the scope of the invention.

### Examples:

### Synthesis of PETh-16:0/18:1-d5 (glycerol-d5) Method I:

### 1,2-Isopropylideneglycerol-d5 (2)

Glycerol-d8 (2.5g, 25.7 mmol) was mixed with 2,2-dimethoxypropane (3.9g, 37.4 mmol) in dry acetone (30mL) and catalytic amount of p-toluenesulfonic acid (pTsOH) (10mg) was added. The resulting solution was stirred for 24 hours at room temperature (being 20°C in the context of this invention). Solvents were evaporated in vacuo to give oily product of 3.62g.

### 1,2-Isopropylidene-glycer-3-yl levulinate-d5 (3)

To a solution of compound **2** (3.6g, 26.2 mmol) in dichloromethane (DCM) (30ml) was added levulinic acid (3.3g, 28.4 mmol) and dimethylaminopyridine (DMAP) (0.64g, 5.29 mmol). N,N'-Dicyclohexylcarbodiimine (DCC) (5.9g, 25.7 mmol) in 20 ml DCM was added to the stirring solution at 0°C dropwise. The reaction mixture was stirred overnight at room temperature. Precipitated dicyclohexyl urea was removed by filtration via short plug of Celite. Solution was concentrated in vacuo, the residue was dissolved in Et₂O, washed with NaHCO₃ sat., H₂O, dried over MgSO₄, filtered and concentrated to give crude product **3,** yield 3.5g.

### 3-Levulinoylglycerol-d5 (4)

3.5g (15.8 mmol) of compound **3** was mixed with 25ml of AcOH/H₂O (3/1). The mixture was heated to 50°C and stirred at this temperature for 16 hours. Solvents were evaporated. The residue was co-evaporated with toluene then acetone. Oily crude product was dissolved in EtOAc, dried over MgSO₄, concentrated to give 1.8g of product **4.**

### 1-Palmitoyl-3-levulinoylglycerol-d5 (5)

To a solution of compound **4** (1.8g, 9.93 mmol) in DCM (30ml) was added palmitic acid (2.37g, 9,24 mmol) and DMAP (0.23g, 1.90 mmol). DCC (1.9, 8.29 mmol, in 10 ml DCM) was added to the stirring solution at 0°C dropwise. The reaction mixture was stirred overnight at room temperature. Precipitated dicyclohexyl urea was removed by filtration via short plug of Celite. Solution was concentrated in vacuo, the residue was dissolved in Et₂O, washed with NaHCO₃ sat., H₂O, dried over MgSO₄, filtered and concentrated to give crude product, which was purified by flash chromatography (silica, toluene/acetone 90/10 to 80/20) to yield pure product **5** 0.75g.
¹HNMR (CDCl₃) 2.80, 2.60 (each t, 4H, COCH₂CH₂CO), 2.35 (t, 2H, CH₂CO), 2.20(s, 3H, OC-CH₃), 1.66-1.61 (m, 2H, CH₂),1.25 (br. s, 24H, CH₂ x 12), 0.87 (t, 3H, CH₃).

### 1-Palmitoyl-2-oleoyl-3-levulinoylglycerol-d5 (6)

To a solution of compound **5** (0.75g, 1.79 mmol) in DCM (30ml) was added oleic acid (0.54g, 1.91 mmol) and DMAP (0.042g, 0.35 mmol). DCC (0.4g, 1.74 mmol, in 10 ml DCM) was added to the stirring solution at 0°C dropwise. The reaction mixture was stirred overnight at room temperature. Precipitated dicyclohexyl urea was removed by filtration via short plug of Celite. Solution was concentrated in vacuo, the residue was dissolved in Et₂O, washed with NaHCO₃ sat., H₂O, dried over MgSO₄, filtered and concentrated to give the product 0.7g.
¹HNMR (CDCl₃) 5.35 (m, 2H, CH=CH), 2.74, 2.58 (each t, 4H, COCH₂CH₂CO), 2.32 (m, 4H, 2xCH₂CO), 2.19 (s, 3H, OC-CH₃), 2.02 (m, 4H, 2x**CH₂**CH=) 1.66-1.61 (m, 4H, 2xCH₂),1.58 (m, 2H, O₂CCH₂**CH₂**-Oleic), 1.25 (m, 42H, CH₂ x 21), 0.88(t, 6H, 2xCH₃).

### 1-Palmitoyl-2-oleoylglycerol-d5 (7)

To the mixture of compound **6** (0.7g, 1.02 mmol) and pyridine (2ml), a solution of hydrazine hydrate (0.17g, 3.4 mmol) in AcOH/pyridine (2.2ml/3.3ml) was added. The reaction mixture was stirred at room temperature for 5 min. 1 ml of 2,4-pentadienone was added and the resulting mixture was stirred for another 5 min. Hexane was added. Two-phase system formed. Hexane emulsion was washed several times with water, dried over MgSO₄ and concentrated in vacuo to give 0.6g of product **7,** pure on thin layer chromatography (TLC) (Toluene/Et₂O 80/20)
¹HNMR (CDCl₃) 5.35 (m, 2H, CH=CH), 2.34 (m, 4H, 2xCH₂CO), 2.02 (m, 4H, 2x**CH₂**CH=) 1.63 (m, 4H, 2xCH₂), 1.26 (m, 44H, CH₂ x 22), 0.88(t, 6H, 2xCH₃).

### 2-Cyanoethoxybis(N,N-diisopropylamino)phosphine (14)

13.75g (8.73 ml, 0.1 mol) of PCl₃ was added to a stirred mixture of dry acetonitrile (100ml) and diisopropylamine (**11,** 60.5g, 84 ml, 0.60 mol) at room temperature over 1 hour. Hexane (100 ml) was added followed by 7.0g (98.5 mmol) of hydroxypropionitrile (**13**) at room temperature. over 30 minutes. Reaction mixture was stirred for 1 hour and filtered through paper filter to remove solids. Hexane layer was collected and concentrated in vacuo to give oily residue (11.0 g) of almost pure product **14.**

### Diisopropylammonium 5-(benzylthio)-tetrazolide (16)

To a solution of 5-benzylthio-1H-tetrazole (**15,** 2.0g, 10.4 mmol, 34.6ml of 0.3M solution in acetonitrile (ACN)) 70 ml of Et₂O was added followed by 1.26g (1.75 ml, 12.45 mmol) of diisopropylamine (**11**). White precipitate formed. Reaction mixture was stirred for 1 hour and product **16** was filtered out, dried in vacuo to give 2.6g of the salt product **16.**

### 2-Cyanoethoxy-(ethoxy)-(N,N-diisopropylamino)phosphine (8)

Ethanol (0.61g, 1eq) was dissolved in 7ml DCM (dry), compound **16** (2.57g, 8.76 mmol) was added to the solution. Compound **14** (4g, 13.3 mmol) was added to the reaction mixture dropwise at room temp. as a solution in dry DCM (4ml). No precipitate formed during reaction. Reaction mixture was stirred for 2 hours at room temperature. Solvents were evaporated in vacuo and the residue was dissolved in EtOAc/NEt₃ (200ml/20ml), washed with sat. NaHCO₃, H₂O, dried over MgSO₄ and concentrated in vacuo to give oily residue which was purified on short silica gel column (Silica, Hexane/EtOAc/NEt₃ 90/10/1), yield 1.91g
¹HNMR (CDCl₃) 3.90-3.92 (m,2H, O**CH₂**CH₂CN) 3.85 (q, 2H, O**CH₂**CH₃), 2.83 (q, 2H, 2xCH), 2.60 (t, 2H, OCH₂**CH₂**CN) 1.25 (t, 3H, O**CH₂**CH₃), 1.06 (d, 12H, 4xCH₃)

### 1-Palmitoyl-2-oleoyl-3-(2-cyanoethoxy)(ethoxy)phosphinyl-glycerol-d5 (9)

Compound **7** (0.6g, 1.0 mmol) and compound **8** (0.22g, 0.89 mmol) were dissolved in dry ACN (70mL) under argon. Solution of tetrazole (0.132g, 1,89 mmol, 4.2ml of 0.45M solution) was added dropwise at room temperature via syringe. The resulting mixture was stirred for 1.5 hours. No precipitation was observed. Di-tert.-butyl peroxide (0.81g, 1.16ml of 70% solution in H₂O) was added and reaction mixture was stirred for 2 hours at room temp. Solvents were evaporated, the residue was dissolved in DCM, washed with NH4CI sat., dried and concentrated in vacuo to give oily residue of 0.65g, which was purified by flash chromatography silica gel column (hexane/acetone 90/10 to 80/20 gradient) to give 0.45g pure product **9**
¹HNMR (CDCl₃) 5.35 (m, 2H, CH=CH), 4.24 (m, 2H, -O**CH₂**CH₂CN), 4.18 (m, 2H,-O**CH₂**CH₃) 2.77 (t, 2H, -OCH₂**CH₂**CN), 2.34 (m, 4H, 2xCH₂CO), 2.02 (m, 4H, 2x**CH₂**CH=) 1.66-1.61 (m, 4H, 2xCH₂),1.25-1.36 (m, 47H, 22xCH₂ , -OCH₂**CH₃**), 0.88(t, 6H, 2xCH₃).

### 1-Palmitoyl-2-oleoyl-3-(ethoxy)(hydroxy)phosphinyl-glycerol-d5 or PEth-16:0/18:1-d5 (glycerol-d5) (10) and its ammonium salt (11, M⁺ = NH4⁺)

Compound **9** (0.45g, 0.59 mmol) was dissolved in dry DCM (20ml) and 1,8-Diazabicyclo[5.4.0]undec-7-ene (DBU) (0.59g, 3.88 mmol) was added to the solution at room temperature under argon. In 5 min reaction was quenched with 0.5ml of AcOH. Solvents were evaporated in vacuo and the residue was purified on silica gel column (DCM/acetone 8/2 first followed by gradient: DCM/acetone/MeOH 7/2/1 to 6/2/2 to 5/2/3) 2 fractions were collected
¹HNMR (CDCl₃) 5.35 (m, 2H, CH=CH), 3.88 (q, 2H, O**CH₂**CH₃), 2.28 (m, 4H, 2xCH₂CO), 2.02 (m, 4H, -**CH₂**CH=CH**CH₂**-) 1.58 (m, 4H, 2x**CH₂**CH₂CO), 1.25-1.33 (m, 47H, 22xCH₂, -OCH₂**CH₃**), 0.88(t, 6H, 2xCH₃).

The PEth acid (10) was dissolved in 5ml of tetrahydrofuran (THF) and 10ml of NH₄OH (30% solution) was added to the solution and left for stirring (16 hours). Reaction mixture was concentrated in vacuo and crude salt was purified on silica gel column impregnated with DCM/toluene/EtOH/NH4OH 79/9/10/2 to 70/8/20/2. TLC (DCM/toluene/MeOH/NH4OH 75/12/12/1) Rf 0.3
M=460mg

### Synthesis of PETH-16:0/18:1-d5 (glycerol-d5) Method II:

### 1,2-O-isopropylidene-3-(4-methoxybenzyl)-glycerol-d5 (17)

A solution of compound **2** (2.44g, 17.8 mmol) in DMF (20 ml) was added dropwise to a suspension of NaH (1.07g, 60% dispersion in mineral oil, 26.7 mmol) in DMF (100ml). The reaction mixture was stirred at room temperature for 30 min., and 4-methoxybenzyl chloride (3.35g, 21.4 mmol) was added. The reaction was continued until compound **2** was totally consumed (disappeared on TLC), water was added slowly to quench the excess NaH. Diethyl ether was added to dilute the reaction mixture, which was then washed with water and brine. The organic phase was dried over MgSO₄ and concentrated. The crude product was purified by flash column chromatography (Silica, hexane/ethyl acetate 4/1) to give 4.01g of product **17.**
¹HNMR (CDCl₃) 7.00 (d, 2H, Ph), 6.91 (d, 2H, Ph), 4.63 (s, 2H, CH₂-Ph), 4.63 (s, 2H, CH₂O-), 4.00 (m, 1H, CH), 3.81 (s, 3H, OCH₃), 1.21 (s, 6H, 2xCH₃C).

### 3-(4-Methoxybenzyl)-glycerol-d5 (18)

To a solution of compound **17** (4.01g, 15.6mmol) in methanol (50 ml) was added p-toluenesulfonic acid monohydrate (260 mg, 1.4 mmol). The reaction mixture was stirred at room temperature for 1-2 hours until compound **17** was totally consumed. NaHCO₃ 240mg) was added and the reaction mixture was concentrated. The residue was purified by flash column chromatography (Silica, hexane/ethyl acetate 1/3) to give 2.5g of product **18.**

### 3-(4-Methoxybenzyl)-1-palmitoyl-glycerol-d5 (19)

To a solution of compound 18 (2.5g, 11.5 mmol), palmitic acid (3.30g, 12.7 mmol) and DMAP (70mg, 0.61 mmol) in DCM (75 ml) was added a solution of DCC (4.52g, 22.0 mmol) in DCM (15 ml) dropwise at 0°C. The reaction mixture was stirred at room temperature overnight under Argon. Dicyclohexylurea was removed by filtering through celite, and the filtrate was evaporated. The residue was purified by flash column chromatography (Silica, hexane/ethyl acetate 1/3) to give product **19** 3.67g.
¹HNMR (CDCl₃) 7.24 (d, 2H, Ph), 6.88 (d, 2H, Ph), 4.48 (s, 2H, CH₂-Ph), 3.80 (s, 3H, OCH₃), 2.31 (t, 2H, CH₂CO), 1.60-1.62 (m, 2H, CH2), 1.25 (br, s, 24H, 12xCH2), 0.87 (t, 3H, CH3).

### 2-(Benzyloxymethyl)-3-(4-methoxybenzyl)-1-palmitoyl-glycerol-d5 (20)

To a solution of compound 19 (3.6g, 7.9 mmol) and di-isopropyl ethylamine (2.28g, 17.5 mmol) in dry DCM (25 ml) at room temperature was added dropwise chloromethyl benzyl ether (2.51g, 16.0 mmol). The resulting mixture was stirred at room temperature overnight, diluted with DCM, washed with saturated NH₄Cl and water, dried over MgSO₄ and concentrated. The crude product mixture was purified by flash column chromatography (Silica, hexane/ethyl acetate 1/5) to afford 4.2 g of product **20** as colorless oil.
¹HNMR (CDCl₃) 7.30-7.24 (m, 2H, Ph), 6.91-7.01 (m, 7H, Ph), 6.02 (s, 2H, -OCH₂O-), 4.68 (s, 2H, CH₂-Ph), 3.80 (s, 3H, OCH₃), 2.32 (t, 2H, CH₂CO), 1.66 (m, 2H, CH2), 1.26 (br, s, 24H, 12xCH2), 0.88 (t, 3H, CH3).

### 2-(Benzyloxymethyl)- 1-palmitoyl-glycerol-d5 (21)

To a solution of compound **20** (4.2g, 7.5 mmol) in DCM/H₂O (20/1, 40 ml) at room temperature was added on one portion 2,3-Dichloro-5,6-dicyano-1,4-benzoquinone (DDQ) (2.25g, 9.87 mmol). The resulting mixture was stirred at room temperature for 30 min, decanted and the precipitate was washed with DCM. The combined organic solution was washed with saturated NaHCO₃ and brine, dried over MaSO₄ and concentrated. The crude product mixture was purified by flash column chromatography (Silica, hexane/ethyl acetate 3/1) to afford 3.76 g of product **21** as viscous oil.
¹HNMR (CDCl₃) 7.30-6.93 (m, 5H, Ph), 6.02 (s, 2H, -OCH₂O-),2.32 (t, 2H, CH₂CO), 1.66 (m, 2H, CH2), 1.26 (br, s, 24H, 12xCH2), 0.88 (t, 3H, CH3).

### O-Ethyl O-[2"-(trimethylsilyl)ethyl] O-(1'-palmitoyl-2'-(benzyloxymethyl)-3'-glyceryl-d5) phosphate (22)

A solution of compound **21** (3.7g, 8.38 mmol) in minimum volume of THF was added to a vigorously stirred solution of (2-(trimethylsilyl)ethyl dichlorophosphite (1.84g, 8.38 mmol) and N,N-diisopropylethylamine (2.57g, 20.0 mmol) in dry, degassed THF (20 ml) at -78°C. The mixture was stirred at the same temperature for 1 hour, ethanol (8.38 mmol) in minimum volume of THF was added slowly. The resulting suspension was stirred for 2 hours at the same temperature, the cooling batch was then removed, and stirring was continued for an additional 1 hour at room temperature. The solvent was removed, and the residue was suspended in EtOAc. The solids were removed by filtration through Celite, and the filtrate was concentrated to provide the crude phosphite triester. The triester obtained was then dissolved in DCM (about 50 ml) at 0°C, H₂O₂ (30%, 19.4 mmol) was added, and the mixture was stirred vigorously for 1 hour at 0°C. The excess oxidizing agent was destroyed by the addition of trimethyl phosphite (2-3 ml). The mixture was diluted with DCM, saturated NaCl was added and the layers were separated. The organic phase was dried over MgSO₄ and concentrated, the crude product was purified by flash column chromatography (Silica, hexane/ethyl acetate 1/1) to afford 4.36 g of product **22** as viscous oil.
¹HNMR (CDCl₃) 7.30-6.93 (m, 5H, Ph), 6.02 (s, 2H, -OCH₂O-),3.99-4.04 (m, 4H,-O**CH₂**CH₃; -O**CH₂**CH₂TMS) 2.32 (t, 2H, CH2CO), 1.66 (m, 2H, CH2), 1.26-1.33 (m, 24H, 12xCH2; 3H, O**CH₂**CH₃), 0.99 (t, 2H, -OCH₂**CH₂**TMS), 0.88 (t, 3H, CH3).

### O-Ethyl O-[2"-(trimethylsilyl)ethyl] O-(1'-palmitoyl-3'-glyceryl-d5) phosphate (23)

To a solution of 22 (4.36g, 6.7 mmol) in absolute ethanol (200ml) at room temperature was added Raney-nickel (ca. 6g), and the resulting suspension was stirred in an atmosphere of H₂ for 16 hours. The H₂ was replaced with argon, Celite (2g) was added, and the mixture was stirred an additional 10 min. The mixture was then filtered, and the filtrate was evaporated to dryness. The crude product was purified by flash column chromatography (Silica, hexane/ethyl acetate 2/1) to afford 3.1 g of product **23** as opaque oil.
¹HNMR (CDCl₃) 3.99-4.04 (m, 4H, -O**CH₂**CH₃; -O**CH₂**CH₂TMS) 2.32 (t, 2H, CH₂CO), 1.66 (m, 2H, CH2), 1.26-1.33 (m, 24H, 12xCH2; 3H, O**CH₂**CH₃), 0.99 (t, 2H,-OCH₂**CH₂**TMS), 0.88 (t, 3H, CH3), 0.06 (s, 9H,TMS)

### O-Ethyl O-[2"-(trimethylsilyl)ethyl] O-(1'-palmitoyl-2'-oleoyl-3'-glyceryl-d5) phosphate (24)

To a solution of 23 (3.1g, 5.7 mmol), oleic acid (1.77g, 6.3 mmol) and DMAP (0,57 mmol) in dry DCM (80 ml) at room temperature was added dropwise a solution of DCC (1.34g, 6.5 mmol) in DCM (40 ml). The resulting suspension was stirred for 6 hours at room temperature, the mixture was filtered through Celite, and the filtrate was evaporated. The crude product was by flash column chromatography (Silica, hexane/ethyl acetate 2/1) to afford 4.1g of product **24** as opaque viscous oil.
¹HNMR (CDCl₃) 5.35 (m, 2H, CH=CH), 3.99-4.04 (m, 4H, -O**CH₂**CH₃; -O**CH₂**CH₂TMS) 2.32-2.35 (m, 4H, 2xCH₂CO), 2.16 (m, 4H, -**CH₂**CH=CH**CH₂**-) 1.66 (m, 4H, 2xCH2), 1.26-1.33 (m, 47H, 12xCH2; O**CH₂**CH₃), 0.99 (t, 2H, -OCH₂**CH₂**TMS), 0.88 (t, 3H, CH3), 0.06 (s, 9H,TMS)

### 1-Palmitoyl-2-oleoyl-3-(ethoxy)(hydroxy)phosphinyl-glycerol-d5 or PEth-16:0/18:1-d5 (glycerol-d5) (10)

To a solution of 24 (4.1g, 5.13 mmol) in DCM (15 ml) at 0°C was added CF₃CO₂H (15 ml), and the mixture was stirred at the same temperature for 30 min, then warmed to room temperature. The solvents were evaporated, the crude product was then repeatedly taken up in toluene (50ml) and the solvent evaporated to remove last traces of CF₃CO₂H before being dried under high vacuum. The product was purified by the same method as in method I to give pure product **10** as free PEth acid. Which can be transferred to salt, ammonium salt product **11** for example, in the same manner as in Method I.

As can be seen from the respective NMR analysis of the respective products given above, the products were pure and no unwanted regioisomers (both 1,2-position reversed position isomer (biologically) and 1,3-position isomer because of chemical acyl-migration) were observed.

## Claims

1. Phosphatidylalkanol homologues having isotopically labelled glycerol moieties.

2. Phosphatidylalkanol homologues according to claim 1, **characterized in that** the alkanol is selected from the group consisting of methanol, ethanol, propanols, butanols, pentanols, hexanols and heptanols, preferably ethanol.

3. Phosphatidylalkanol homologues according to claim 1 or 2, **characterized in that**
a) one, two, three, four or five hydrogen atoms, particularly five, of the glycerol moiety are replaced with deuterium, or
b) one, two or three, particularly three, carbon atoms of the glycerol moiety are replaced by ¹³C-istoptes, or
c) one, two, three, four or five hydrogen atoms, particularly five, of the glycerol moiety are replaced with deuterium, and one two or three, particularly three, carbon atoms of the glycerol moiety are replaced by ¹³C-istoptes
or,
d) one or more hydrogen atoms of the fatty acid side chains are replaced by deuterium, or
e) one to eighteen carbon atoms in the fatty acid side chains are replaced by ¹³C-isotopes,
or,
f) any combination of the above described labelling is present.

4. Phosphatidylalkanol homologues according to claim 1, 2 or 3, **characterized in that** they are represented by either of the two general formulae 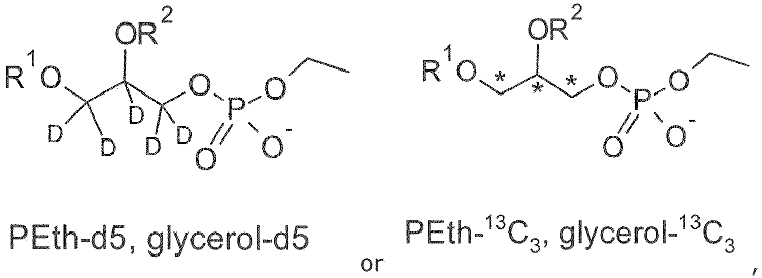 or further by either of the four general formulae 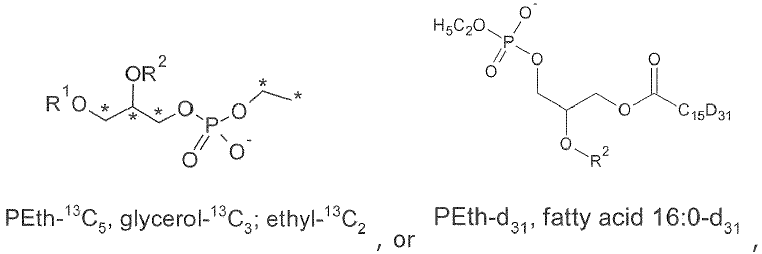 or 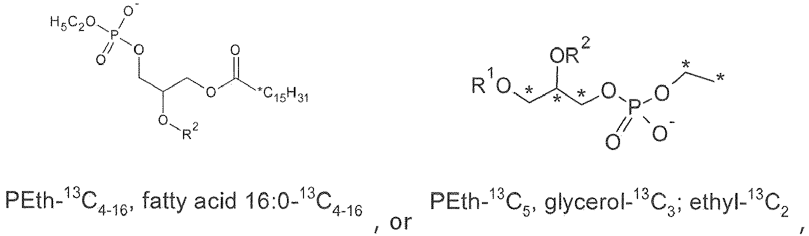 wherein D is deuterium, the asterisk denotes a ¹³C-atom and R¹ and R² are, independently from one another, acyl residues having C14 to C22 saturated alkyl chains or C14 to C22 unsaturated alkyl chains with up to seven double bonds.

5. Phosphatidylalkanol homologues according to claim 1, 2, or 3, **characterized in that** they are represented by
the general formula A 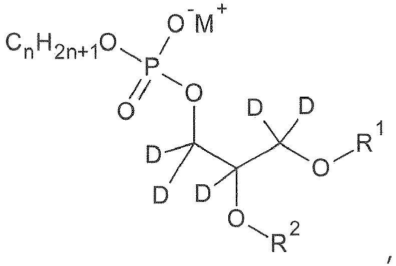 or the general formula B 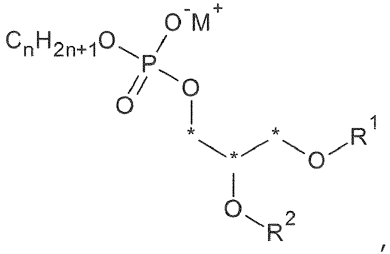 or further by the general formula C 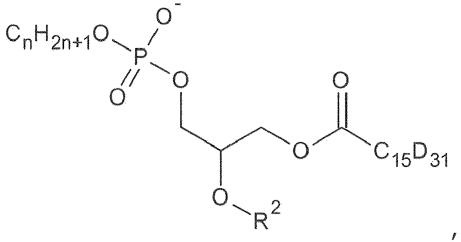 or further by the general formula D 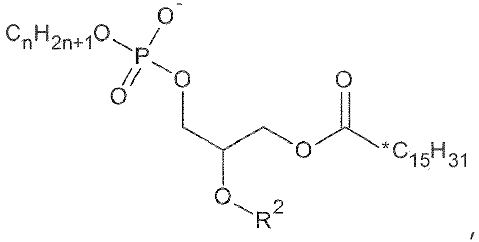 wherein R¹ and R² have the same meaning as in claim 4 and are fatty acid chains with different number of carbons and different number of double bonds, and M is H, NH4, Na, K, N(CH₃)₃⁺ and the asterisk (*) represents an ¹³C-atom, preferably R¹ is a saturated alkyl chain and R² is an unsaturated alkenyl chain.

6. Phosphatidylalkanol homologues according to claim 4 or 5, selected from the group consisting of 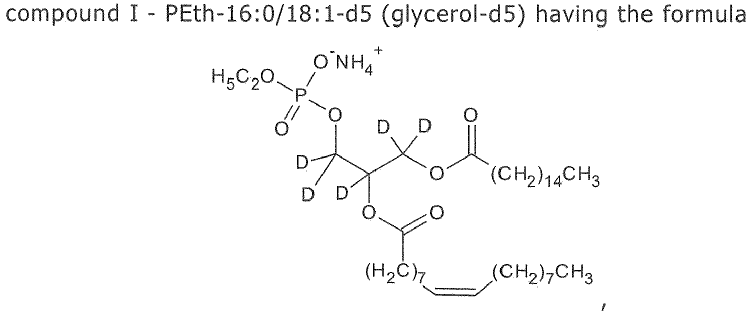 or 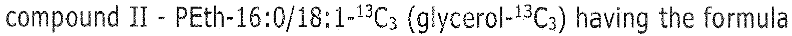 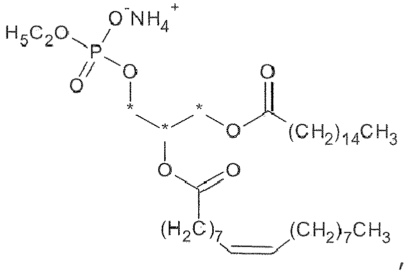 or 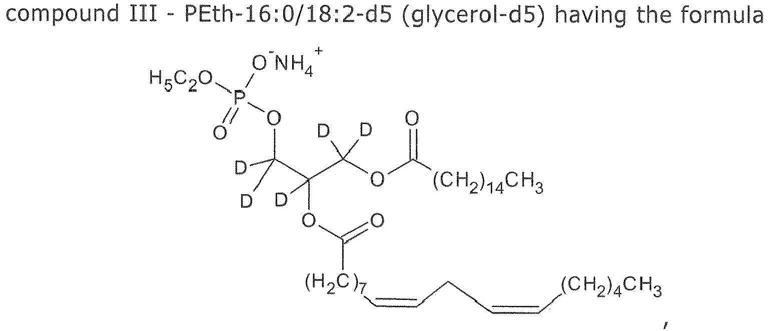 or 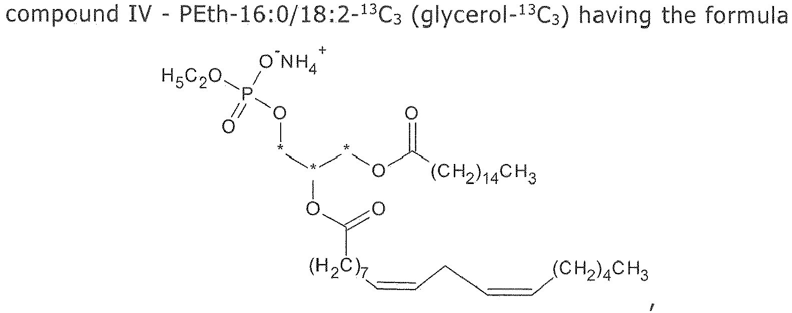 or

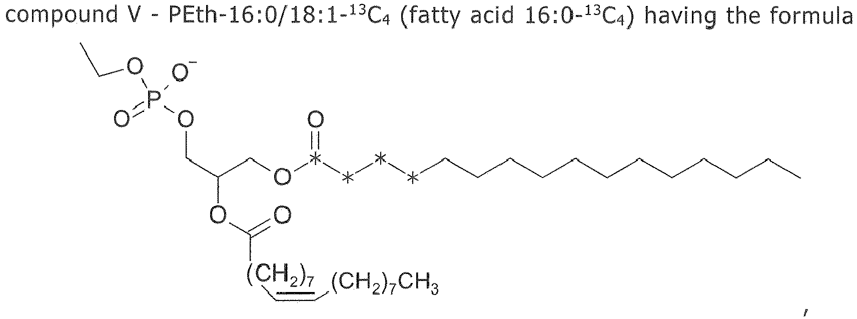

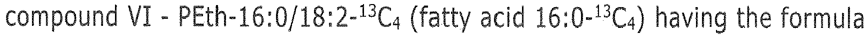

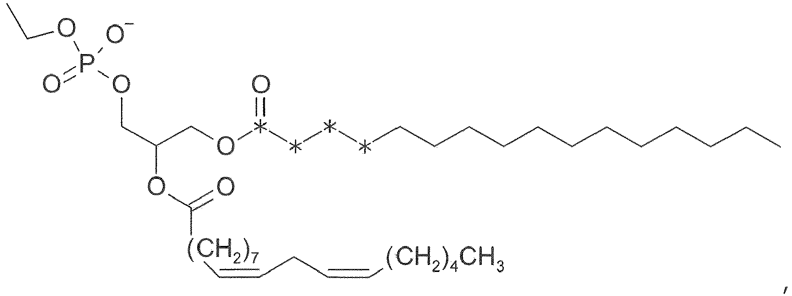

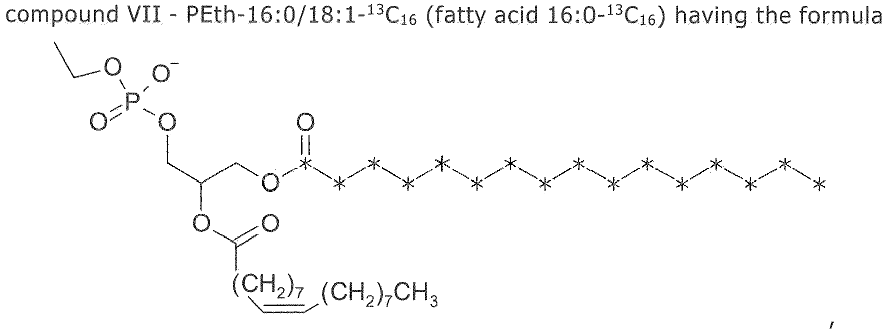

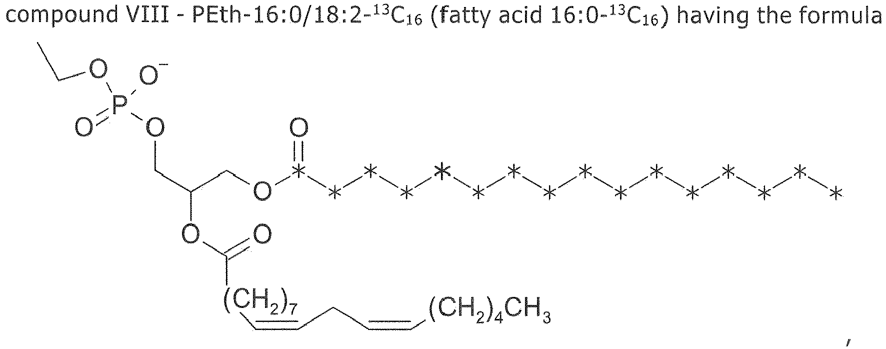

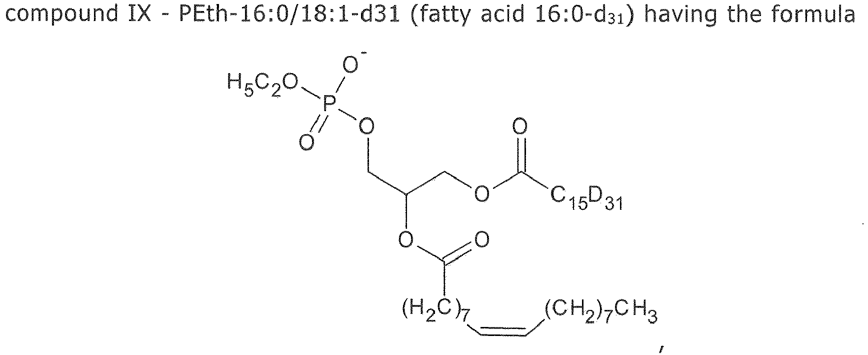

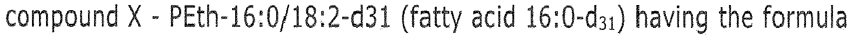

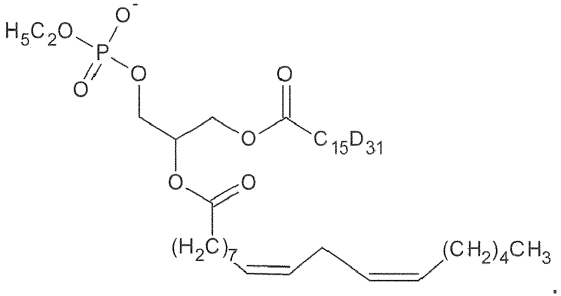

7. Phosphatidylalkanol homologues I, II, II or IV according to claim 6, **characterized in that** instead of ammonia as cation, the cation is selected from the group consisting of Na⁺, K⁺ and N(CH₃)₃⁺, or homologues V to X according to claim 6, **characterized in that** the cation is selected from the group consisting of ammonia, Na⁺, K⁺ and N(CH₃)₃⁺.

8. Method for the preparation of isotopically labelled phosphatidylalkanol homologues, comprising or consisting of the following steps:
mI(1) preparation of isotopically labelled 1-aklyoyl-2-alkenoylglycerol comprising or consisting of the steps
mIa1) providing labelled glycerol in which some or all, preferably all, hydrogen atoms have been replaced with deuterium, or
mIa2) providing labelled glycerol in which some or all, preferably all, carbon atoms have been replaced with ¹³C-isotopes, or
mIa3) providing labelled glycerol in which some or all, preferably all, hydrogen atoms have been replaced with deuterium, and some or all, preferably all, carbon atoms have been replaced with ¹³C-isotopes; or
mIa4) providing unlabelled glycerol;
mIb) protecting two of the OH/OD-groups of the glycerol moiety, preferably under formation of an acetal-structure;
mIc) protecting the remaining OH/OD-group in the 3-position with a protecting group, preferably a levulinoyl-group;
mId) deprotecting the first two OH/OD-groups;
mIe) addition of a C12 to C18 saturated alkyl chain, preferably C15 to C17 saturated alkyl chain, especially preferred C16 saturated alkyl chain to the sn-1 position of the glycerol moiety;
mIf) addition of a mono-, di-, tri- and tetra-unsaturated chain, preferably C16 to C20 mono-, di-, tri- and tetra-unsaturated chain, more preferred mono- and di-unsaturated chain, especially preferred are C18:1 or C18:2 to the sn-2 position of the glycerol moiety;
mIg) removing the second protective group, preferably with hydrazine hydrate;
mI(2) reacting the isotopically labelled 1-alkyloyl-2-alkenoylglycerol product of step (1) with a phosphorylation reagent having
i) if an unlabelled glycerol is provided in step mIa4), a labelled alkanol group, preferably a labelled ethanol group; wherein all or some of the hydrogen atoms of the alkanol residue are replaced by deuterium and/or wherein some or all the carbon atoms of the alkanol residue are replaced by ¹³C-isotopes or
a labelled fatty acid chain, wherein all or some of the hydrogen atoms are replaced by deuterium and/or wherein some or all the carbon atoms of the alkanol residue are replaced by ¹³C-isotopes;
ii) if a labelled glycerol is provided in steps mIa1), mIa2) or mIa3), an unlabelled alkanol group, preferably an unlabelled ethanol group, wherein no atom of the alkanol residue has been replaced by deuterium and/or ¹³C;
mIh) optionally removing a protecting agent, if present on the phosphorylation agent;
mIi) optionally converting the product to a salt-form of the product.

9. Method for the preparation of isotopically labelled phosphatidylalkanol homologues, comprising or consisting the following steps:
mIIa1) providing labelled glycerol in which some or all, preferably all, hydrogen atoms have been replaced with deuterium, or
mIIa2) providing labelled glycerol in which some or all, preferably all, carbon atoms have been replaced with ¹³C-isotopes, or
mIIa3) providing labelled glycerol in which some or all, preferably all, hydrogen atoms have been replaced with deuterium, and some or all, preferably all, carbon atoms have been replaced with ¹³C-isotopes; or mIIa4) providing unlabelled glycerol;
mIIb) protecting two of the OH/OD-groups of the glycerol moiety, preferably under formation of an acetal-structure;
mIIc) protecting the remaining OH/OD-group in the 3-position with a protecting group, preferably 4-methoxybenzyl;
mIId) deprotecting the first two OH/OD-groups;
mIIe) addition of a C12 to C18 saturated alkyl chain, preferably C15 to C17 saturated alkyl chain, especially preferred C16 saturated alkyl chain to the sn-1 position of the glycerol moiety, or
addition of a labelled C12 to C18 saturated alkyl chain, preferred C16 and C18 saturated alkyl chain to the sn-1 position of the glycerol moiety;
mIIf) protecting the hydroxyl-group in 2-position, preferably with benzyloxylmethyl or tetrahydroxy pyranyl;
mIIg) removal of the first protection group;
mIIh) coupling the protected isotopically labelled protected 1-acylglyerol product of step mIIg) with a phosphorylation reagent having
i) if an unlabelled glycerol is provided in step mIa4), a labelled alkanol group, preferably a labelled ethanol group; wherein all or some of the hydrogen atoms of the alkanol residue are replaced by deuterium and/or wherein some or all the carbon atoms of the alkanol residue are replaced by ¹³C-isotopes
or
a labelled fatty acid, preferably 16:0 and 18:0, is introduced to the sn-1 position;
ii) if a labelled glycerol is provided in steps mIa1), mIa2) or mIa3), an unlabelled alkanol group, preferably an unlabelled ethanol group, wherein no atom of the alkanol residue has been replaced by deuterium and/or ¹³C;
mIIi) deprotecting the hydroxyl group in 2-position;
mIIj) addition of a mono-, di-, tri- and tetra-unsaturated chain, preferably C16 to C20 mono-, di-, tri- and tetra-unsaturated chain, more preferred mono- and di-unsaturated chain, especially preferred are C18:1 or C18:2 to the sn-2 position of the glycerol moiety;
mIIh) optionally removing a protecting agent, if present on the phosphorylation agent;
mIIi) optionally converting the product to a salt-form of the product.

10. Method according to claim 8 or 9, **characterized in that** the phosphorylation agent is selected from the group consisting of alkyl dichlorophosphite, benzyl dichlorophosphite and compound 8, (trimethylsilyl)ethyl dichlorophosphite and compound **8.**

11. Method for the preparation of a compound of general formula 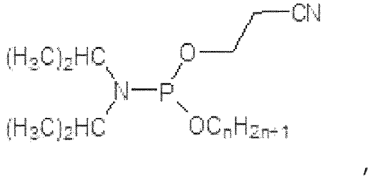 in particular of the following formula 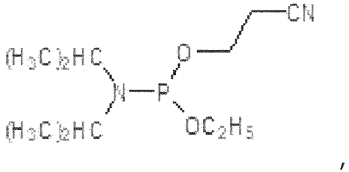 comprising or consisting of the steps
aa) providing diisopropylamine and reacting with 2-cyanoethanol and phosphorous trichloride;
bb) providing diisopropylamine and reacting with 5-Benzylthio-1H-tetrazole;
cc) reacting the product from step aa) with an alkanol, in particular ethanol, in the presence of the product of step bb).

12. Use of phosphatidylalkanol homologues according to any of claims 1 to 7, particularly compounds I, II, III and IV, as labelled standards, particularly internal standards, for analytical processes and methods, especially for GC/MS or LC-MS/MS or LC-ESI-MS/MS or CID-OzID MS or HPLC or LT-MS or HPLC or high throughput UPLC^{®}-MSMS.

13. Use of phosphatidylalkanol homologues prepared according to any of claims 8 to 10, particularly those with isotopically labelled glycerol moieties, as labelled standards, particularly internal standards, for analytical processes and methods, especially for GC/MS or LC-MS/MS or LC-ESI-MS/MS or CID-OzID MS or HPLC or LT-MS or HPLC or high throughput UPLC^{®}-MSMS.

14. Use of phosphatidylalkanol homologues according to any of claims 1 to 7, particularly compounds I, II, III, IV, V, VI, VII, and VIII or phosphatidylalkanol homologues prepared according to any of claims 8 to 10, particularly those with isotopically labelled glycerol moieties, as reference materials and internal standards for quantitative analysis of PEth homologues in human blood samples, especially by LC-MS/MS or LC-ESI-MS/MS, or for the study of individual PEth homologues.

15. Use of phosphatidylalkanol homologues according to any of claims 1 to 7, particularly compounds I, II, III, IV, V, VI, VII, VIII, IX, and X or phosphatidylalkanol homologues prepared according to any of claims 8 to 10, particularly those with isotopically labelled glycerol moieties, as reference materials and internal standards for quantitative analysis of PEth homologues in human blood samples, especially by LC-MS/MS or LC-ESI-MS/MS, or for the study of individual PEth homologues using the internal calibration method (Multipoint Internal Calibration Method) with a mixture of at least three internal standards.
